# EUROPÄISCHE PATENTSCHRIFT

(11) **EP 0 562 512 B1**
(45) Veröffentlichungstag und Bekanntmachung des Hinweises auf die Patenterteilung: **21.02.2001**
(21) Anmeldenummer: 93104658.5
(22) Anmeldetag: 22.03.1993
(51) Int. Cl.: C07D 213/82, C07D 213/81, C07D 213/89, C07D 401/12, C07D 409/12

(54) **Sulfonamidocarbonylpyridin-2-carbonsäureamide und ihre Verwendung als Arzneimittel**
Sulfonamidocarbonylpyridine-2-carboxamides and their use as medicines
Sulfonamidocarbonylpyridine-2-carboxamides et leur utilisation comme médicaments

(30) Priorität: 24.03.1992 DE 4209424; 14.11.1992 DE 4238506
(43) Veröffentlichungstag der Anmeldung: 29.09.1993
(73) Patentinhaber: HOECHST AKTIENGESELLSCHAFT, 65926 Frankfurt am Main (DE)
(72) Erfinder: Weidmann, Klaus, Dr., W-6242 Kronberg/Ts. (DE); Bickel, Martin, Dr., W-6380 Bad Homburg (DE); Günzler-Pukall, Volkmar, Dr., W-3550 Marburg (DE)

(56) Entgegenhaltungen:
- EP-A- 0 176 741
- EP-A- 0 278 453
- EP-A- 0 278 454
- EP-A- 0 414 216
- EP-A- 0 463 592

## Beschreibung

Die Erfindung betrifft Sulfonamidocarbonylpyridin-2-carbonsäureamide sowie ihre Verwendung als Arzneimittel gegen fibrotische Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazallulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

Es bestand nunmehr die Aufgabe nach Verbindungen zu suchen, die stärker antifibrotisch sind als die bisher bekannten Verbindungen.

Gelöst wird die Aufgabe durch das Bereitstellen von Sulfonamidocarbonylpyridin-2-carbonsäureamide der allgemeinen Formel I, 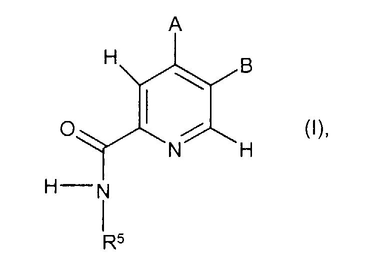 worin
- A =: H und B = -CO-NR⁶-R⁷ oder
- B =: H und A = -CO-NR⁶R⁷ bedeutet
- R⁶: Wasserstoff oder ein 1 oder 2-wertiges physiologisch verwendbares Kation,
- R⁷: einen Rest der Formel II bedeutet

-SO₂-C-U-D-W (II),
in welchem
- C: eine Bindung oder (C₁-C₄)-Alkandiyl,
- U: eine Bindung oder -O- bedeutet,
- D: xeine Bindung oder (C₁-C₄)-Alkandiyl,
- W: einen Phenylrest bedeutet, wobei U nur dann in der Bedeutung von -O- steht, wenn C nicht eine Bindung bedeutet oder wenn D nicht eine Bindung bedeutet und
- W: substituiert ist durch Wasserstoff, Fluor, Chlor, (C₁-C₆)-Alkyl, (C₃-C₈)-Cycloalkyl, (C₆-C₁₂)-Aryl, (C₇-C₁₆)-Aralkyl, C₃-C₁₂)-Alkenyl, (C₁-C₆)-Alkoxy, Phenoxy, -O-(-CH₂]_{X}C_{f}H_{(2f+1-g})F_{g},
Carbamoyl, N-(C₁-C₁₀)-Alkylcarbamoyl, N,N-Di(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-phenylcarbamoyl, N-(C₁-C₈)-Alkyl-N-(C₇-C₁₁)-Phenylalkylcarbamoyl, N-(C₁-C₈)-Alkyl-N-Phenylalkylcarbamoyl, N-((C₁-C₁₀)-Alkoxy-(C₁-C₈)alkyl)carbamoyl, N-Phenoxycarbamoyl, N-((C₇-C₁₆)-Phenylalkyloxy-(C₁-C₈)alkyl)carbamoyl, N-(C₁-C₈)-Alkyl-N-(C₁-C₆)-Alkoxy-(C₁-C₈)alkyl)carbamoyl, N-(C₁-C₈)-Alkyl-N-(Phenoxy-(C₁-C₈)alkyl)carbamoyl, N-(C₁-C₈)-Alkyl-N-((C₇-C₁₆)-Phenylalkyloxy-(C₁-C₈)alkyl)carbamoyl,
(C₁-C₈)-Akanoylamino, (C₃-C₈)-Cycloalkanoylamino, Phenylamino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₈)-alkyl, (C₃-C₈)-Cycloalkanoylamino-(C₁-C₈)alkyl, Phenylamino-(C₁-C₈)-alkyl, (C₇-C₁₁)-Phenylalkanoylamino-(C₁-C₈)-alkyl,
wobei die Reste, die einen Arylrest enthalten, ihrerseits am Aryl substituiert sein können mit 1, 2 oder 3 gleichen oder verschiedenen Substituenten aus der Reihe Hydroxy, Carboxyl, (C₁-C₄)-Alkoxy, Phenoxy, Benzyloxy,
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)-Alkylcarbonyloxy, (C₃-C₈)-Cycloalkylcarbonyloxy, Benzoyloxy, (C₇-C₁₁)-Phenylalkylcarbonyloxy,
(C₁-C₈)-Alkoxycarbonyloxy, Phenoxycarbonyloxy, (C₇-C₁₁)-Phenalkyloxycarbonyl, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy-(C₁-C₄)-alkyl-carbamoyl, Acyloxy-(C₁-C₄)-alkylcarbamoyl,
Carbamoyloxy, N-(C₁-C₆)-Alkylcarbamoyloxy, N,N-Di(C₁-C₆)-alkylcarbamoyloxy, N-(C₃-C₈)-Cycloalkylcarbamoyloxy,
- R⁵: einen unverzweigten (C₁-C₄)-Alkylrest bedeutet, der mit einem Rest aus der Reihe Hydroxy, Carboxyl, (C₁-C₄)-Alkoxy, Phenoxy, Benzyloxy,
(C₁-C₉)-Alkoxycarbonyl, Phenoxycarbonyl, (C₇-C₁₁)-Phenylalkyloxycarbonyl, (C₃-C₈)-Cycloalkoxycarbonyl,
(C₁-C₁₂)-Alkylcarbonyloxy, (C₃-C₈)-Cycloalkylcarbonyloxy, Benzoyloxy, (C₇-C₁₁)-Phenylalkylcarbonyloxy,
(C₁-C₈)-Alkoxycarbonyloxy, Phenoxycarbonyloxy, (C₇-C₁₁)-Phenylalkylcarbonyloxy, (C₃-C₈)-Cycloalkoxycarbonyloxy,
Carbamoyl, N-(C₁-C₆)-Alkylcarbamoyl, N,N-Di(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy-(C₁-C₄)-alkyl-carbamoyl, Acyloxy-(C₁-C₄)-alkyl-carbamoyl, (C₁-C₆)-Alkoxy-(C₁-C₆)-alkylcarbamoyl,
Carbamoyloxy, N-(C₁-C₆)-Alkylcarbamoyloxy, N,N-Di(C₁-C₆)alkylcarbamoyloxy, N-(C₃-C₈)-Cycloalkylcarbamoyloxy, oder mit
einem Rest der allgemeinen Formel III

-CO-P (III),
worin
- P: ein über seine terminale Aminogruppe gebundenes Di- oder Tripeptid oder ein über ihre Aminogruppe gebundenes Aminosäure-Derivat bedeutet, substituiert ist
- f: 1 bis 5
- g: 0,1 bis (2f+1) und
- x: 0 oder 1
bedeuten.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen, insbesondere der Leber.

Schließlich betrifft die Erfindung die Verbindungen der allgemeinen Formel I zur Verwendung als Arzneimittel.

Insbesondere betrifft die Erfindung Verbindungen der Formel I zur Anwendung als Fibrosuppressiva und gegen fibrotische Erkrankungen und zur Behandlung von Störungen des Stoffwechsels von Collagen und collagenähnlichen Stoffen.

Die Erfindung betrifft ebenso ein Arzneimittel enthaltend Verbindungen der allgemeinen Formel I und ggf. einen pharmazeutisch verträglichen Träger.

Verbindungen der Formel I, wurden hergestellt, indem
i) die Pyridin-2-carbonsäure-Derivate bzw. die entsprechenden Ester der Formel 11 mit den Aminen der Formel 5 umgesetzt wurden, oder
ii) die Pyridin-5-carbonsäure-Derivate der Formel 12 mit den SulfonamidDerivaten der Formel 9 umgesetzt wurden, oder
iii) die Pyridin-5-carbonsäureamid-Derivate der Formel 13 mit den Sulfonsäure-Derivaten der Formel 2 umgesetzt wurden, vgl. Schema 2,
wobei die Verbindungen der Formeln 12 bzw. 13 ihrerseits aus den Verbindungen der Formel 7 nach den bekannten Methoden hergestellt wurden.

Schema 2 verdeutlicht die Herstellung von Verbindungen der Formel Ia.

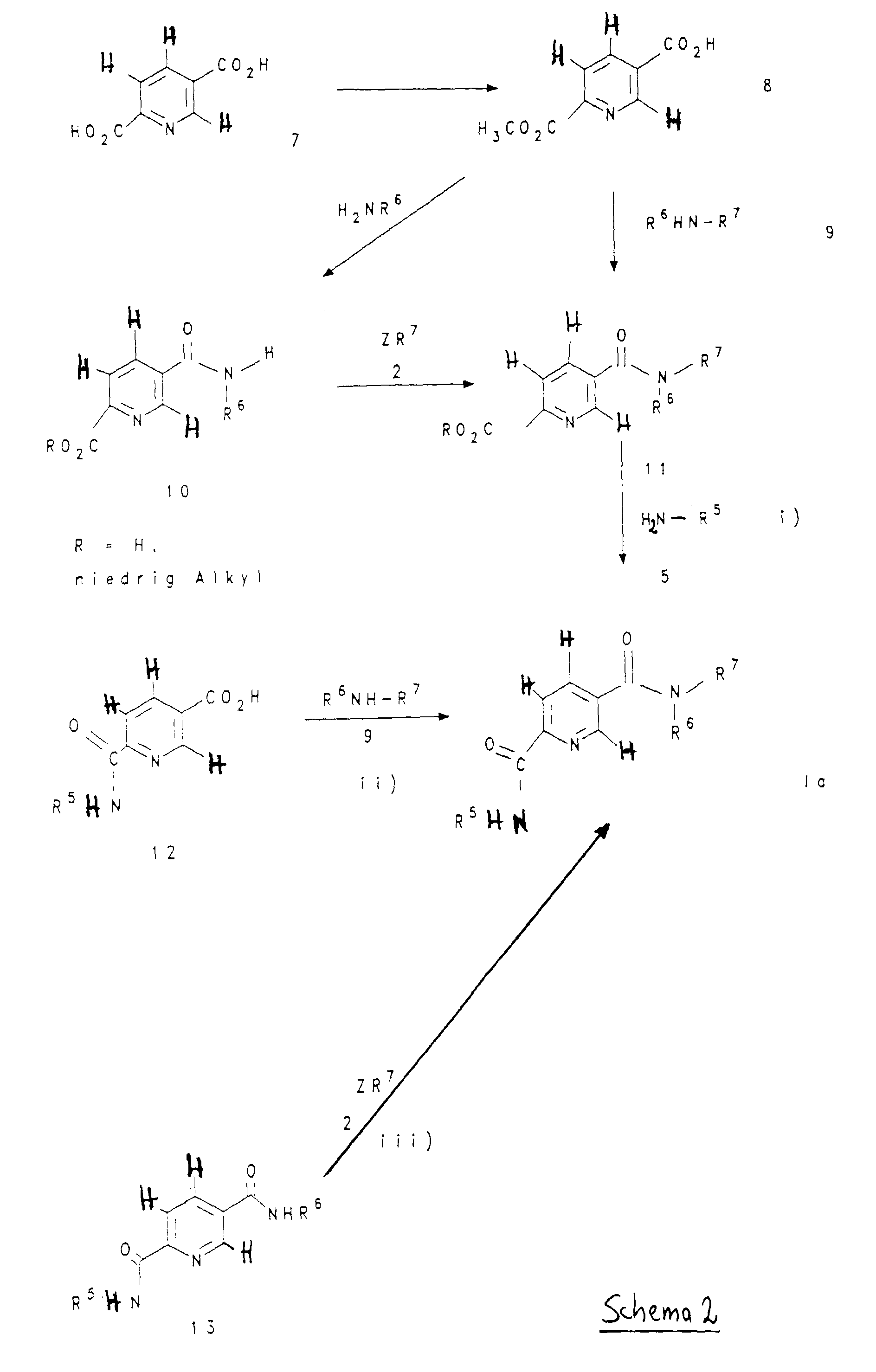

Entsprechend CA: Vol 68, 1968, 68840 h können aus den substituierten Pyridin-2,5-dicarbonsäuren der Formel 7 unter Veresterungsbedingungen die Pyridin-2-carbonsäureester-5-carboxylate der Formel 8 hergestellt werden. Geeignete Bedingungen sind z. B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Herstellung der Verbindungen der Formel 11 erfolgt aus den Verbindungen der Formel 8 und den Sulfonamidderivaten der Formel 9 wobei es zweckmäßig sein kann, beide Reaktanden mit Hilfsreagenzien zu aktivieren (Houben-Weyl: Methoden der Organischen Chemie, Band IX, Kapitel 19, Seiten 636-637).

An Reagenzien zur Carbonsäurereaktivierung können die dem Fachmann bekannten Substanzen, wie Thionylchlorid, Oxalylchlorid, Pivaloylchlorid oder Chlorameisensäureester-Derivate Verwendung finden. Es ist nicht immer notwendig, diese aktivierten Derivate der Verbindungen der Formel 8 zu isolieren. Meist ist es zweckmäßig, sie nach Herstellung in situ oder als Rohprodukt mit den Sulfonamidderivaten der Formel 9 umzusetzen.

Zweckmäßigerweise werden die Verbindungen der Formel 9 zunächst mit einer anorganischen oder organischen Base, wie z. B. Natrium- oder Kaliumhydroxid, -carbonat, -alkoxid, -hydrid, -amid, Ammoniak, Triethylamin, Tributylamin, Pyridin bei -20° bis + 150° C, vorzugsweise bei 0° - 80° C zur Reaktion gebracht und dieses Reaktionsgemisch mit einer Verbindung der Formel 8 oder dessen aktivierter Form umgesetzt. Die Umsetzung erfolgt in einem inerten Lösungsmittel, wie z.B. Methylenchlorid, Methanol, Ethanol, Aceton, Essigsäureethylester, Toluol, Tetrahydrofuran, Acetonitril, N,N-Dimethylformamid, N,N-Dimethylacetatamid, Nitromethan, Dimethylsulfoxid oder Gemischen dieser Lösungsmittel. Alternativ können die Ester der Formel 11 mit Hilfe der üblichen Kondensationsreagenzien (wie z.B. N,N'-Dicyclohexylcarbodiimid/4-N,N-Dimethylaminopyridin) hergestellt werden.

Die Reaktion der Pyridin-2-carbonsäureester 11 mit Aminen HNH R⁵ führt zu den erfindungsgemäßen Verbindungen der Formel Ia.

Alternativ können zur Herstellung der Verbindungen der Formel Ia die Verbindungen 11 (R = niederes Alkyl) zu den Pyridin-2-carbonsäure-Derivaten 11 (R = H) verseift werden und diese anschließend mit den Aminen HNH R⁵ nach den üblichen Methoden der Peptidchemie zu den erfindungsgemäßen Verbindungen der Formel Ia gekuppelt werden.

Die oben beschriebene Reaktionsfolge gemäß Schema 2 zur Herstellung von in 5-Stellung eine Carbonylsulfonamidgruppe aufweisenden Verbindungen der Formeln Ia läßt sich auch zur Herstellung solcher Verbindungen anwenden, die eine Carbonylsulfonamidgruppe in 4-Stellung aufweisen. Begonnen wird dann mit Verbindungen der allgemeinen Formel 7': 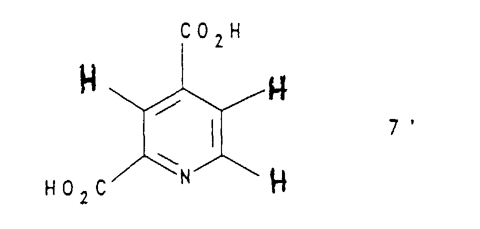 Verbindungen der Formel I, in denen R⁵ einen Hydroxyalkylrest, einen Alkoxyalkylrest oder ein Derivat hiervon bedeuten, wurden aus den entsprechend substituierten Sulfonamiden der Formel 9 und Pyridin-2-carbonsäuremethylester-4(bzw. 5)-carbonsäure und anschließender Aminolyse der Verbindungen der Formel 11 (R = niederes Alkyl); Schema 2) mit den entsprechenden Aminen hergestellt.

Zur Herstellung der Verbindungen der Formel I, in denen NH R⁵ einen Glycyl-Rest bedeutet, wurden die Verbindungen der Formel 11 (R = niederes Alkyl) zu den Pyridin-2-carbonsäure-Derivaten der Formel 11 (R = H) verseift und diese mit den entsprechenden Glycinderivaten kondensiert. Die freien Glycylamide, in denen NH R⁵ NH CH₂ CO₂H bedeutet, wurden durch Verseifung der (Glycylester)amide oder durch katalytische Hydrierung der Glycylbenzylester erhalten.

Zur Herstellung von Verbindungen gemäß der allgemeinen Formel I (Ia) nach dem Schema 2 werden Verbindungen eingesetzt, in denen R⁶ Wasserstoff bedeutet. Die Salzbildung, nach der R⁶ ein physiologisch verwendbares Kation bedeutet, erfolgt bevorzugt anschließend. Als Salzbildner kommen bevorzugt N-Alkylamine, (Hydroxyalkyl)amine und (Alkoxyalkyl)amine, wie z.B. 2-Ethanolamin, 3-Propanolamin, 2-Methoxyethylamin, 2-Ethoxyethylamin und α,α,α-Tris-(hydroxymethyl)methylamin (= Trispuffer, Tromethan) oder auch basische Aminosäuren, wie z.B. Histidin, Arginin und Lysin in Frage.

Die erfindungsgemäßen Verbindungen der Formel I besitzen wertvolle pharmakologische Eigenschaften und zeigen insbesondere antifibrotische Wirksamkeit.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben- analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z. B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z. B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimitel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägestoffe für die gewünschte Arzneimittelformulierung sind beispielsweise neben Lösemitteln, Gelbildner, Suppositoriengrundlagen, Tabletten-Hilfsstoffen und anderen Wirkstoffträgern auch Antioxidantien, Dispergiermittel, Emulgatoren, Entschäumer, Geschmachskorrigentien, Konservierungsmittel, Lösungsvermittler oder Farbstoffe.

Die folgenden Beispiele sollen die Erfindung erläutern.

### Beispiel 1

5-[((4-Methoxyphenylsulfonyl)amino)-carbonyl]-pyridin-2-carbonsäure-(2-hydroxyethyl)amid
a) Pyridin-2-carbonsäuremethylester-5-carbonsäurechlorid
   14,5 g (80 mmol) Pyridin-2-carbonsäure-methylester-5-carbonsäure werden in 200 ml wasserfreiem Toluol mit 6,48 ml Thionylchlorid und 2 ml wasserfreiem N,N-Dimethylformamid versetzt. Unter Rühren wird 3 h auf 70° C erhitzt. Danach engt man in Vakuum ein und löst den Rückstand in 150 ml Tetrahydrofuran.
b) 5-[(4-Methoxyphenylsulfonyl)amino)-carbonyl]pyridin-2-carbonsäure-methylester
   Zu 16,45 g (88 mmol) 4-Methoxybenzolsulfonsäure-amid in 200 ml Tetrahydrofuran gibt man bei 0° C 19,75 g (176 mmol) Kalium-tert. Butylat. Nachdem 3 h bei Raumtemperatur gerührt wurde, gibt man die Lösung aus Beispiel 1a) bei 0.5° C zu. Man rührt 3 h unter Erwärmung auf Raumtemperatur, gibt 300 ml Ethylacetat zu, extrahiert zweimal mit wäßriger NaHCO₃-Lösung, säuert die wäßrige Phase mit konzentrierter wäßriger Salzsäure an, extrahiert 3 mal mit Dichlormethan, trocknet, engt ein, kristallisiert den Rückstand aus Methanol und erhält 9,9 g farbloses, kristallines Produkt, Fp. 197-199° C.
c) 2,1 g (6 mmol) der vorstehenden Verbindung werden in 10 ml Ethanolamin gelöst und 5 h bei 50° C gerührt. Man gibt 40 ml Wasser hinzu, säuert unter Eiskühlung (5-10° C) mit conc. HCl an, saugt den ausgefallenen Feststoff ab und wäscht diesen mehrfach mit Wasser. Man erhält 1,96 g der Titelverbindung in Form farbloser Kristalle, Fp. 221-223° C.

### Beispiel 2

5-[(4-Methoxyphenylsulfonyl)amino-carbonyl]-pyridin-2-carbonsaure-(glycylmethylester)amid
a) 5-[(4-Methoxyphenylsulfonyl)amino-carbonyl]-pyridin-2-carbonsäure
   3,0 g (8,6 mmol) der Verbindung aus Beispiel 1b) werden in 100 ml Methanol gelöst und bei 0-5° C mit 17,2 ml (17,2 mmol) 1 N NaOH versetzt. Nachdem 4 h bei Raumtemperatur gerührt wurde, engt man in Vakuum ein, nimmt den Rückstand in Wasser auf und gibt bei 0-5° C 17,2 mol (17,2 mmol) 1 N HCI zu, saugt den Feststoff ab, wäscht mehrfach mit Wasser und erhält 2,58 g der obigen Verbindung, Fp. 234-236° C.
b) Zu 1,81 g (5,4 mmol) der obigen Verbindung in 25 ml wasserfreiem Tetrahydrofuran gibt man bei 0° C 1,8 g (5,94 mmol) Triethylamin, rührt 20 min bei dieser Temperatur, tropft dann 0,71 g (5,94 mmol) Pivaloylchlorid zu und rührt 3 h bei 0° C. Dann gibt man 0,75 g (5,98 mmol) Glycinmethylester-Hydrochlorid zu, rührt 3 h bei 0° C, läßt dann auf 20° C erwärmen und über Nacht stehen. Zur Reaktionslösung gibt man 2N HCI und extrahiert dreimal mit Dichlormethan, trocknet, engt ein, chromatographiert den Rückstand mit Ethylacetat/Methanol (4:1) an Kieselgel, engt entsprechende Fraktionen ein, kristallisiert aus Diisopropylether um und erhält 1,44 g der Titelverbindung, Fp. 150-152° C.

### Beispiel 3

5-[((Phenylsulfonyl)amino)carbonyl]-pyridin-2-carbonsäure-(2-hydroxyethyl)amid
a) 5-[((Phenylsulfonyl)amino)carbonyl]pyridin-2-carbonsäuremethylester
   Die Verbindung wird analog Beispiel 1b) aus 3,46 g (22 mmol) Benzolsulfonsäureamid 2,46 g (22 mmol) Kalium-tert. Butylat und 4,0 g (20 mmol) Pyridin-2-carbonsäure-methylester-5-carbonsäurechlorid erhalten. Man erhält nach dem Umkristallisieren aus Methanol 1,6 g Produkt, Fp. 197-198°C.
b) Analog Beispiel 1c) wird die Titelverbindung aus 5-[((Phenylsulfonyl)amino)carbonyl-pyridin-2-carbonsäure-methylester und Ethanolamin als farblose, kristalline Substanz erhalten, Fp. 249-250° C.

### Beispiel 8

5-[((4-n-Butoxyphenylsulfonyl)amino)carbonyl]-pyridin-2-carbonsäure-(2-hydroxyethyl)amid
a) 4-n-Butoxybenzolsulfonsäureamid
   Zu 10 g 4-n-Butoxybenzolsulfonsäure-chlorid werden unter Eiskühlung 100 ml methanolische Ammoniaklösung getropft. Nach 1/2 Stunde Rühren bei 20° C wird eingeengt, mit Wasser versetzt, angesäuert auf pH 1-2 und abgesaugt, Fp. 99-101° C.
b) 5-[((4-n-Butoxyphenylsulfonyl)amino)carbonyl]-pyridin-2-carbonsäure-methylester Analog Beispiel 1b) werden 4,6 g (20 mmol) vorstehender Verbindung, 2,5 g (22 mmol) Kalium-tert.butylat und 5,0 g (25 mmol) Pyridin-2-carbonsäuremethylester-5-carbonsäure-chlorid umgesetzt. Ausgefallenes Kaliumsalz wird im Dioxan-/Wasser-Gemisch mit 2 N HCI angesäuert. Das ausgefallene Produkt wird abgesaugt und getrocknet; Ausbeute 1,5 g; Fp. 174-176° C.
c) Die Titelverbindung erhält man aus 0,2 g (0,51 mmol) obiger Verbindung und 5 ml Aminoethanol bei 80° C (1 h). Man versetzt mit Wasser, säuert an, saugt ab, trocknet und erhält 0,19 g farblose kristalline Substanz, Fp. 176-178°C.

### Beispiel 9

5-[((4-Trifluormethoxyphenylsulfonyl)amino)carbonyl]-pyridin-2-carbonsäure-(2-hydroxyethyl)amid
a) 4-Trifluormethoxybenzolsulfonsäureamid wird aus dem entsprechenden Sulfonsäurechlorid durch Reaktion mit methanolischer Ammoniak-Lösung erhalten. Das Rohprodukt wird mit Wasser versetzt, angesäuert, abgesaugt und getrocknet, Fp. 143-145° C.
b) 5-[((4-Trifluormethoxyphenylsulfonyl)amino)carbonyl]-pyridin-2-carbonsäuremethylester.
   Analog Beispiel 1b) setzt man 4,8 g (20 mmol) der vorstehenden Verbindung, 2,5 g (22 mmol) Kalium-tert.butylat in Dioxan und 5 g (25 mmol) Pyridin-2-carbonsäuremethylester-5-carbonsäurechlorid um. Nach Einengen wird der Rückstand in Wasser aufgenommen, angesäuert, die Fällung abgesaugt und getrocknet; 3,4 g Rohprodukt (Fp. 210-214° C), das aus 75 ml Ethylacetat umkristallisiert wird, 1,5 g farblos kristalline Substanz, Fp. 221-223° C.
c) Aus 0,6 g (ca. 1,5 mmol) vorstehender Verbindung und 5 ml Aminoethanol erhält man nach einstündigem Erhitzen auf 80° C, Abkühlen, Versetzen mit Wasser, Ansäuern mit halbkonzentrierter HCI, Extraktion mit Ethylacetat, Trocknen, Einengen und Kristallisation mit Diethylether die Titelverbindung, Fp. 202-204° C.

### Beispiel 10

5-[((2,5-Bis-[1,1,1-trifluorethyloxy]phenylsulfonyl)amino)carbonyl]-pyridin-2-carbonsäure-(2-hydroxyethyl)amid

Die Titelverbindung wird analog Beispiel 1c) aus 5-[((2,5-Bis-[1,1,1-trifluorethyloxy]phenylsulfonyl)amino)carbonyl]-pyridin-2-carbonsäuremethylester und Aminoethanol erhalten. Man bringt mit Diethylether zur Kristallisation, Fp. 190-192°C.

### Beispiel 11

5-[((4-[2,2,2-Trifluorethyloxy]phenylsulfonyl)amino)carbonyl]-pyridin-2-carbonsäure(2-hydroxyethyl)amid

### Beispiel 12

5-[((4-[2,2,2-Trifluorethyloxy]phenylsulfonyl)amino)carbonyl]-pyridin-2-carbonsäure(3-hydroxypropyl)amid

### Beispiel 13

5-[((4-[2,2,2-Trifluorethyloxy]phenylsulfonyl)amino)carbonyl]-pyridin-2-carbonsäure(2-methoxyethyl)amid

### Beispiel 14

5-[((4-[2,2,2-Trifluorethyloxy]phenylsulfonyl)amino)carbonyl]-pyridin-2-carbonsäure(glycylmethylester)amid

### Beispiel 15

5-[((4-[2,2,2-Trifluorethyloxy]phenylsulfonyl)amino)carbonyl]-pyridin-2-carbonsäureglycylamid

### Beispiel 16

5-[((4-[2,2,2-Trifluorethyloxy]phenylsulfonyl)amino)carbonyl]-pyridin-2-carbonsäure-(2-hydroxyethylamino)glycylamid

### Beispiel 17

5-[((4-[2,2,3,3,3-Pentafluorpropyloxy]phenylsulfonyl)amino)carbonyl]-pyridin-2-carbonsäure-(2-hydroxyethyl)amid

### Beispiel 18

5-[((4-[2,2,3,3,3-Pentafluorpropyloxy]phenylsulfonyl)amino)carbonyl]-pyridin-2-carbonsäure-(2-methoxyethyl)amid

### Beispiel 23

5-[((4-[2,2,3,3,4,4,4-Heptafluorbutyloxy]phenylsulfonyl)amino)carbonyl]-pyridin-2-carbonsäure-(2-ethoxyethyl)amid

### Beispiel 24

5-[((4-[2,2,3,3,4,4,4-Heptafluorbutyloxy]phenylsulfonyl)amino)carbonyl]-pyridin-2-carbonsäure-(glyclymethylester)amid

### Beispiel 25

5-[((4-Phenoxy-phenylsulfonyl)amino)carbonyl]-pyridin-2-carbonsäure-2- (hydroxyethyl)amid

### Beispiel 26

5-[((4-Phenoxy-phenylsulfonyl)amino)carbonyl]-pyridin-2-carbonsäure-(2-ethoxyethyl)amid

### Beispiel 27

5-[((4-Phenoxy-phenylsulfonyl)amino)carbonyl]-pyridin-2-carbonsäure-(2-methoxyethyl)amid

### Beispiel 28

5-[((2-Phenyl-phenylsulfonyl)amino)carbonyl]-pyridin-2-carbonsäure-(2-hydroxyethyl)amid

### Beispiel 29

5-[((2-Phenyl-phenylsulfonyl)amino)carbonyl]-pyridin-2-carbonsäure-(glycylmethylester)amid

### Beispiel 33

5-[((4-[3-(Trifluormethyl)phenoxy]phenylsulfonyl)amino)carbonyl]-pyridin-2-carbonsäure-(2-hydroxyethyl)amid
Fp. 167-168°C (aus Wasser)

### Beispiel 34

5-[((4-[3-(Trifluormethyl)phenyloxy]phenylsulfonyl)amino)carbonyl]-pyridin-2-carbonsäure-(glycylmethylester)amid

### Beispiel 40

5-[((Phenylmethylsulfonyl)amino)carbonyl]-pyridin-2-carbonsäure-(2,-hydroxyethyl)amid

### Beispiel 41

5-[((2,-Phenylethylsulfonyl)amino)carbonyl]-pyridin-2-carbonsäure-,hydroxyethyl)amid

### Beispiel 42

5-[((2-(4-Fluorphenyl)ethylsulfonyl)amino)carbonyl-pyridin-2,-carbonsäure-(2-hydroxyethyl)amid

### Beispiel 43

5-[((2-(4-Methoxyphenyl)ethylsulfonyl)amino)carbonyl]-pyridin-2,-carbonsäure-(2-hydroxyethyl)amid

### Beispiel 44

5-[((3-Phenyl-n-propylsulfonyl)amino)carbonyl]-pyridin-2,-carbonsäure-(2-hydroxyethyl)amid

### Beispiel 45

5-[((4-Phenyl-n-butylsulfonyl)amino)carbonyl]-pyridin-2,-carbonsäure-(2-hydroxyethyl)amid

### Beispiel 46

5-[((2-Phenoxyethylsulfonyl)amino)carbonyl]-pyridin-2-carbonsäure-(2-hydroxyethyl)amid

### Beispiel 47

5-[((2-(4-Fluorphenoxy)ethylsulfonyl)amino)carbonyl]-pyridin-2,-carbonsäure-(2-hydroxyethyl)amid

### Beispiel 48

5-[((Phenylmethylsulfonyl)amino)carbonyl]-pyridin-2-carbonsäure-(2-methoxyethyl)amid

### Beispiel 49

5-[((2-Phenylethylsulfonyl)amino)carbonyl]-pyridin-2-carbonsäure-(2-methoxyethyl)amid

### Beispiel 50

5-[((2-(4-Fluorphenyl)ethylsulfonyl)amino)carbonyl]-pyridin-2-carbonsäure-(2-methoxyethyl)amid

### Beispiel 51

5-[((2-(4-Methoxyphenyl)ethylsulfonyl)amino)carbonyl]-pyridin-2-carbonsäure-(2-methoxyethyl)amid

### Beispiel 52

5-[((3-Phenyl-n-propylsulfonyl)amino)carbonyl]-pyridin-2-carbonsäure-(2-methoxyethyl)amid

### Beispiel 53

5-[((4-Phenyl-n-butylsulfonyl)amino)carbonyl]-pyridin-2-carbonsäure-(2-methoxyethyl)amid

### Beispiel 54

5-[((2-Phenoxyethylsulfonyl)amino)carbonyl]-pyridin-2-carbonsäure-(2-methoxyethyl)amid

### Beispiel 55

5-[((2-(4-Fluorphenoxy)ethylsulfonyl)amino)carbonyl]-pyridin-2-carbonsäure-(2-methoxyethyl)amid

### Beispiel 56

5-[((Phenylmethylsulfonyl)amino)carbonyl]-pyridin-2-carbonsäure-(glycyl-2-propylester)amid

### Beispiel 57

5-[((2-Phenylethylsulfonyl)amino)carbonyl]-pyridin-2-carbonsäure-(glycyl-2-propylester)amid

### Beispiel 58

5-[((2,-(4-Fluorphenyl)ethylsulfonyl)amino)carbonyl]-pyridin-2-carbonsäure-(glycyl-2-propylester)amid

### Beispiel 59

5-[((2-(4-Methoxyphenyl)ethylsulfonyl)amino)carbonyl]-pyridin-2-carbonsäure-(glycyl-2-propylester)amid

### Beispiel 60

5-[((3-Phenyl-n-propylsulfonyl)amino)carbonyl]-pyridin-2-carbonsäure-(glycyl-2-propylester)amid

Aus Pyridin-2-carbonsäuremethylester-4-carbonsäurechlorid (bzw. 4-carbonsäure) wurden analog hergestellt:

### Beispiel 61

4-[((4-[2,2,2-Trifluorethyloxy]phenylsulfonyl)amino)carbonyl]-pyridin-2-carbonsäure-(2-hydroxyethyl)amid

### Beispiel 62

4-[((4-[2,2,2-Trifluorethyloxy]phenylsulfonyl)amino)carbonyl]-pyridin-2-carbonsäure-(2-methoxyethyl)amid

### Beispiel 63

4-[((4-[2,2,2-Trifluorethyloxy]phenylsulfonyl)amino)carbonyl]-pyridin-2-carbonsäure(glycylmethylester)amid

### Beispiel 64

4-[((4-Phenoxy-phenylsulfonyl)amino)carbonyl]-pyridin-2-carbonsäure-2-(hydroxyethyl)amid

### Beispiel 65

4-[((4-Phenoxy-phenylsulfonyl)amino)carbonyl]-pyridin-2-carbonsäure-(2-ethoxyethyl)amid

### Beispiel 66

4-[((2-Phenyl-phenylsulfonyl)amino)carbonyl]-pyridin-2-carbonsäure(2-hydroxyethyl)amid

### Beispiel 67

4-[((2-Phenyl-phenylsulfonyl)amino)carbonyl]-pyridin-2-carbonsäure(glycylmethylester)amid

### Beispiel 69

4-[((n-Butylsulfonyl)amino)carbonyl]-pyridin-2-carbonsäure-(2-methoxyethyl)amid

### Beispiel 70

4-[((n-Butylsulfonyl)amino)carbonyl]-pyridin-2-carbonsäure-(glycylmethylester)amid

Durch Aminolyse des Esters aus Beispiel 3a) mit den entsprechenden Aminen wurden im Sinne der Erfindung folgende Verbindungen erhalten:

### Beispiel 109

5-[((Phenylsulfonyl)amino)carbonyl]-pyridin-2-carbonsäure-(2-methoxyethyl)amid Fp. 190-191 °C (aus Wasser)

### Beispiel 110

5-[((Phenylsulfonyl)amino)carbonyl]-pyridin-2-carbonsäure-(glycylmethylester)amid Fp. 125-126°C (aus Wasser)

### Beispiel 111

5-[((Phenylsulfonyl)amino)carbonyl]-pyridin-2-carbonsäure-glycylamid Fp. 190-191 °C (aus Wasser)

### Beispiel 112

5-[((4-((2-Phenylethyl)aminocarbonyl)phenylsulfonyl)amino)carbonyl]-pyridin-2-carbonsäure-(2-hydroxyethyl)amid
a) 4-((2-Phenylethyl)aminocarbonyl)benzol-sulfonsäureamid
   20,1 g (0,1 mol) 4-Carboxy-benzolsulfonsäureamid wurden in 300 ml wasserfreiem Tetrahydrofuran suspendiert und bei 0° C unter Rühren tropfenweise mit 15,2 ml (0,11 mol) Triethylamin versetzt. Nach 30 min tropfte man bei 0° C 10,5 ml (0,11 mol) Chlorameisensäureethylester zu, rührte 1 h bei dieser Temperatur, kühlte ab auf -10° C und tropfte 12,1 g (0,1 mol, 12,5 ml) 2-Phenylethylamin in 30 ml wasserfreiem Tetrahydrofuran zu. Nach 1 h bei 0° C erwärmte man auf 20° C, engte im Vakuum ein, behandelte den festen Rückstand mit Wasser, saugte ab, kristallisierte aus Ethanol um und erhielt 20,4 g Produkt, Fp. 243-245° C.
b) 5-[((4-((2-Phenylethyl)aminocarbonyl)phenylsulfonyl)amino)carbonyl]-pyridin-2-carbonsäure-methylester
   1,8 g (10 mmol) Pyridin-2,5-dicarbonsäure-2-methylester wurden in 300 ml wasserfreiem Acetonitril suspendiert und bei 20° C unter Rühren mit 3,0 g (10 mmol) der vorstehenden Verbindung, 2,1 g (10 mmol) N,N'-Dicyclohexylcarbodiimid und 1,2 g (10 mmol) 4-N,N-Dimethylaminopyridin versetzt und 20 h bei 20° C gerührt. Dann wurde vom Ungelösten abfiltriert, das Filtrat im Vakuum eingeengt, der Rückstand in 200 ml Dichlormethan aufgenommen, zweimal mit gesättigter wäßriger NaHCO₃-Lösung, sodann mit 100 ml 2 N wäßriger HCI extrahiert. Die kristalline Fällung wurde anschließend mit warmem Methanol behandelt, abgesaugt und getrocknet. Man erhielt 2,2 g des Esters, Fp. 228-230° C.
c) Die Titelverbindung wurde erhalten, indem 0,8 g (1,7 mmol) der vorstehenden Verbindung in 10 ml 2-Aminoethanol 1 h bei 80 bis 90° C gerührt wurden. Das überschüssige Z-Aminoethanol wurde im Vakuum abdestilliert, der Rückstand in wenig Wasser aufgenommen, mit wäßriger HCI auf pH 1 angesäuert. Das kristalline Produkt wurde nochmals mit Ethylacetat behandelt, abgesaugt und getrocknet: 0,73 g Produkt als farblose Kristalle, Fp. 228-230° C.

### Beispiel 113

5-[((4-Benzylaminocarbonyl)phenylsulfonyl)amino)carbonyl]-pyridin-2-carbonsäure(2-hydroxyethyl)amid

### Beispiel 114

5-[((4-((3-Phenyl-n-propyl)aminocarbonyl)phenylsulfonyl)amino)carbonyl]-pyridin-2-carbonsäure-(2-hydroxyethyl)amid

### Beispiel 115

5-[((4-((4-Phenyl-n-butyl)aminocarbonyl)phenylsulfonyl)amino)carbonyl]-pyridin-2-carbonsäure-(2-hydroxyethyl)amid

### Beispiel 116

5-[((4-((2-(4-Methoxyphenyl)ethyl)aminocarbonyl)phenylsulfonyl)amino)carbonyl]pyridin-2-carbonsäure-(2-hydroxyethyl)amid
Fp. 243-244° C (aus Wasser)

### Beispiel 117

5-[((4-((2-(3,4-Dimethoxyphenyl)ethyl)aminocarbonyl)phenylsulfonyl)amino) carbonyl]-pyridin-2-carbonsäure-(2-hydroxyethyl)amid
Fp. 201-203° C (Ethylacetat)

### Beispiel 118

5-[((4-((2-(2-Methoxyphenyl)ethyl)aminocarbonyl)phenylsulfonyl)amino)carbonyl]pyridin-2-carbonsäure-(2-hydroxyethyl)amid

### Beispiel 119

5-[((4-((2-(4-Fluorphenyl)ethyl)aminocarbonyl)phenylsulfonyl)amino)carbonyl]pyridin-2-carbonsäure-(2-hydroxyethyl)amid

### Beispiel 120

5-[((4-((2-(4-Chlorphenyl)ethyl)aminocarbonyl)phenylsulfonyl)amino)carbonyl]pyridin-2-carbonsäure-(2-hydroxyethyl)amid

### Beispiel 121

5-[((4-(Ethylaminocarbonyl)phenylsulfonyl)amino)carbonyl]-pyridin-2-carbonsäure(2-hydroxyethyl)amid

### Beispiel 122

5-[((4-(n-Butylaminocarbonyl)phenylsulfonyl)amino)carbonyl]-pyridin-2-carbonsäure(2-hydroxyethyl)amid
Fp. 245° C

### Beispiel 123

5-[((4-(n-Hexylaminocarbonyl)phenylsulfonyl)amino)carbonyl]-pyridin-2-carbonsäure(2-hydroxyethyl)amid

### Beispiel 124

5-[((4-(N,N-Di-n-butylaminocarbonyl)phenylsulfonyl)amino)carbonyl]-pyridin-2-carbonsäure-(2-hydroxyethyl)amid

### Beispiel 125

5-[((4-(Cyclohexylaminocarbonyl)phenylsulfonyl)amino)carbonyl]-pyridin-2-carbonsäure-(2-hydroxyethyl)amid

### Beispiel 126

5-[((4 -(2-Methoxyethyl)aminocarbonyl)phenylsulfonyl)amino)carbonyl]-pyridin-2-carbonsäure-(2-hydroxyethyl)amid

### Beispiel 127

5-[((4-((2-Ethoxyethyl)aminocarbonyl)phenylsulfonyl)amino)carbonyl]-pyridin-2-carbonsäure-(2-hydroxyethyl)amid

### Beispiel 128

5-[((4-((3-Ethoxypropyl)aminocarbonyl)phenylsulfonyl)amino)carbonyl]-pyridin-2-carbonsäure-(2-hydroxyethyl)amid

### Beispiel 129

5-[((4-((2-Phenoxyethyl)aminocarbonyl)phenylsulfonyl)amino)carbonyl]-pyridin-2-carbonsäure-(2-hydroxyethyl)amid

### Beispiel 130

5-[((3-(Benzylaminocarbonyl)phenylsulfonyl)amino)carbonyl]-pyridin-2-carbonsäure-(2-hydroxyethyl)amid

### Beispiel 131

5-[((3-((3-Phenylpropyl)aminocarbonyl)phenylsulfonyl)amino)carbonyl]-pyridin-2-carbonsäure-(2-hydroxyethyl)amid

### Beispiel 132

5-[((3-((4-Phenyl-n-butyl)aminocarbonyl)phenylsulfonyl)amino)carbonyl]-pyridin-2-carbonsäure-(2-hydroxyethyl)amid

### Beispiel 133

5-[((3-((2-(4-Methoxyphenyl)ethyl)aminocarbonyl)phenylsulfonyl)amino)carbonyl]pyridin-2-carbonsäure-(2-hydroxyethyl)amid

### Beispiel 134

5-[((3-((2-(3,4-Dimethoxyphenyl)ethyl)aminocarbonyl)phenylsulfonyl) amino)carbonyl]-pyridin-2-carbonsäure-(2-hydroxyethyl)amid

### Beispiel 135

5-[((3-((2-(2-Methoxyphenyl)ethyl)aminocarbonyl)phenylsulfonyl)amino)carbonyl]pyridin-2-carbonsäure-(2-hydroxyethyl)amid

### Beispiel 136

5-[((3-((2-(4-Fluorphenyl)ethyl)aminocarbonyl)phenylsulfonyl)amino)carbonyl]pyridin-2-carbonsäure-(2-hydroxyethyl)amid

### Beispiel 137

5-[((3-((2-(4-Chlorphenyl)ethyl)aminocarbonyl)phenylsulfonyl)amino)carbonyl]pyridin-2-carbonsäure-(2-hydroxyethyl)amid

### Beispiel 138

5-[((3-(Ethylaminocarbonyl)phenylsulfonyl)amino)carbonyl]-pyridin-2-carbonsäure(2-hydroxyethyl)amid

### Beispiel 139

5-[((3-(n-Butylaminocarbonyl)phenylsulfonyl)amino)carbonyl]-pyridin-2-carbonsäure-(2-hydroxyethyl)amid

### Beispiel 140

5-[((3-(n-Hexylaminocarbonyl)phenylsulfonyl)amino)carbonyl]-pyridin-2-carbonsäure-(2-hydroxyethyl)amid

### Beispiel 141

5-[((3-(N,N-Di-n-butylaminocarbonyl)phenylsulfonyl)amino)carbonyl]-pyridin-2-carbonsäure-(2-hydroxyethyl)amid

### Beispiel 142

5-[((3-(Cyclohexylaminocarbonyl)phenylsulfonyl)amino)carbonyl]-pyridin-2-carbonsäure-(2-hydroxyethyl)amid

### Beispiel 143

5-[((3-((2-Methoxyethyl)aminocarbonyl)phenylsulfonyl)amino)carbonyl]-pyridin-2-carbonsäure-(2-hydroxyethyl)amid

### Beispiel 144

5-[((3-((2-Ethoxyethyl)aminocarbonyl)phenylsulfonyl)amino)carbonyl]-pyridin-2-carbonsäure-(2-hydroxyethyl)amid

### Beispiel 145

5-[((3-((3-Methoxypropyl)aminocarbonyl)phenylsulfonyl)amino)carbonyl]-pyridin-2-carbonsäure-(2-hydroxyethyl)amid

### Beispiel 146

5-[((3-((2-Phenylethyl)aminocarbonyl)phenylsulfonyl)amino)carbonyl]-pyridin-2-carbonsäure-(2-hydroxyethyl)amid
F. 189-191 °C

### Beispiel 147

5-[((3-((2-Phenoxyethyl)aminocarbonyl)phenylsulfonyl)amino)carbonyl]-pyridin-2-carbonsäure-(2-hydroxyethyl)amid

### Beispiel 148

5-[((4-(Benzylaminocarbonyl)phenylsulfonyl)amino)carbonyl]-pyridin-2-carbonsäure-(2-methoxyethyl)amid

### Beispiel 149

5-[((4-((3-Phenyl-n-propyl)aminocarbonyl)phenylsulfonyl)amino)carbonyl]-pyridin-2-carbonsäure-(2-methoxyethyl)amid

### Beispiel 150

5-[((4-((4-Phenyl-n-butyl)aminocarbonyl)phenylsulfonyl)amino)carbonyl]-pyridin-2-carbonsäure-(2-methoxyethyl)amid

### Beispiel 151

5-[((4-((2-(4-Methoxyphenyl)ethyl)aminocarbonyl)phenylsulfonyl)amino)carbonyl]-pyridin-2-carbonsäure-(2-methoxyethyl)amid

### Beispiel 152

5-[((4-((2-(3,4-Dimethoxyphenyl)ethyl)aminocarbonyl)phenylsulfonyl)amino) carbonyl]-pyridin-2-carbonsäure-(2-methoxyethyl)amid
Fp. 161-163° C (Ethylacetat)

### Beispiel 153

5-[((4-((2-(2-Methoxyphenyl)ethyl)aminocarbonyl)phenylsulfonyl)amino)carbonyl]-pyridin-2-carbonsäure-(2-methoxyethyl)amid

### Beispiel 154

5-[((4-((2-(4-Fluorphenyl)ethyl)aminocarbonyl)phenylsulfonyl)amino)carbonyl]-pyridin-2-carbonsäure-2-(methoxyethyl)amid

### Beispiel 155

5-[((4-((2-(4-Chlorphenyl)ethyl)aminocarbonyl)phenylsulfonyl)amino)carbonyl]-pyridin-2-carbonsäure-(2-methoxyethyl)amid

### Beispiel 156

5-[((4-(Ethylaminocarbonyl)phenylsulfonyl)amino)carbonyl]-pyridin-2-carbonsäure-(2-methoxyethyl)amid

### Beispiel 157

5-[((4-(n-Butylaminocarbonyl)phenylsulfonyl)amino)carbonyl]-pyridin-2-carbonsäure-(2-methoxyethyl)amid

### Beispiel 158

5-[((4-(n-Hexylaminocarbonyl)phenylsulfonyl)amino)carbonyl]-pyridin-2-carbonsäure-(2-methoxyethyl)amid

### Beispiel 159

5-[((4-(N,N-Di-n-butylaminocarbonyl)phenylsulfonyl)amino)carbonyl]-pyridin-2-carbonsäure-(2-methoxyethyl)amid

### Beispiel 160

5-[((4-(Cyclohexylaminocarbonyl)phenylsulfonyl)amino)carbonyl]-pyridin-2-carbonsäure-(2-methoxyethyl)amid

### Beispiel 161

5-[((4-((2-Methoxyethyl)aminocarbonyl)phenylsulfonyl)amino)carbonyl]-pyridin-2-carbonsäure-(2-methoxyethyl)amid

### Beispiel 162

5-[((4-((2-Ethoxyethyl)aminocarbonyl)phenylsulfonyl)amino)carbonyl]-pyridin-2-carbonsäure-(2-methoxyethyl)amid

### Beispiel 163

5-[((4-((3-Ethoxypropyl)aminocarbonyl)phenylsulfonyl)amino)carbonyl]-pyridin-2-carbonsäure-(2-methoxyethyl)amid

### Beispiel 164

5-[((4-((2-Phenylethyl)aminocarbonyl)phenylsulfonyl)amino)carbonyl]-pyridin-2-carbonsäure-(2-methoxymethyl)amid

### Beispiel 165

5-[((4-((2-Phenoxyethyl)aminocarbonyl)phenylsulfonyl)amino)carbonyl]-pyridin-2-carbonsäure-(2-methoxymethyl)amid

### Beispiel 166

5-[((3-(Benzylaminocarbonyl)phenylsulfonyl)amino)carbonyl]-pyridin-2-carbonsäure-(2-methoxyethyl)amid

### Beispiel 167

5-[((3-((Phenylpropyl)aminocarbonyl)phenylsulfonyl)amino)carbonyl]-pyridin-2-carbonsäure-(2-methoxyethyl)amid

### Beispiel 168

5-[((3-Phenyl-n-butyl)aminocarbonyl)phenylsulfonyl)amino)carbonyl]-pyridin-2-carbonsäure- (2-methoxyethyl)amid

### Beispiel 169

5-[((3-((2-(3,4-Dimethoxyphenyl)ethyl)aminocarbonyl)phenylsulfonyl)amino) carbonyl]-pyridin-2-carbonsäure-(2-methoxyethyl)amid

### Beispiel 170

5-[((3-((2-(3-Methoxyphenyl)ethyl)aminocarbonyl)phenylsulfonyl)amino)carbonyl]-pyridin-2-carbonsäure-(2-methoxyethyl)amid

### Beispiel 171

5-[((3-((2-(2-Methoxyphenyl)ethyl)aminocarbonyl)phenylsulfonyl)amino)carbonyl]-pyridin-2-carbonsäure-(2-methoxyethyl)amid

### Beispiel 172

5-[((3-((2-(4-Fluorphenyl)ethyl)aminocarbonyl)phenylsulfonyl)amino)carbonyl]-pyridin-2-carbonsäure-2-(methoxyethyl)amid

### Beispiel 173

5[((3-((2-(4-Chlorphenyl)ethyl)aminocarbonyl)phenylsulfonyl)amino)carbonyl]-pyridin-2-carbonsäure-(2-methoxyethyl)amid

### Beispiel 174

5-[((3-(Ethylaminocarbonyl)phenylsulfonyl)amino)carbonyl]-pyridin-2-carbonsäure-(2-methoxyethyl)amid

### Beispiel 175

5-[((3-(n-Butylaminocarbonyl)phenylsulfonyl)amino)carbonyl]-pyridin-2-carbonsäure-(2-methoxyethyl)amid

### Beispiel 176

5-[((3-(n-Hexylaminocarbonyl)phenylsulfonyl)amino)carbonyl]-pyridin-2-carbonsäure-(2-methoxyethyl)amid

### Beispiel 177

5-[((3-(N,N-Di-n-butylaminocarbonyl)phenylsulfonyl)amino)carbonyl]-pyridin-2-carbonsäure-(2-methoxyethyl)amid

### Beispiel 178

5-[((3-(Cyclohexylaminocarbonyl)phenylsulfonyl)amino)carbonyl]-pyridin-2-carbonsäure-(2-methoxyethyl)amid

### Beispiel 179

5-[((3-((2-Methoxyethyl)aminocarbonyl)phenylsulfonyl)amino)carbonyl]-pyridin-2-carbonsäure-(2-methoxyethyl)amid

### Beispiel 180

5-[((3-((2-Ethoxyethyl)aminocarbonyl)phenylsulfonyl)amino)carbonyl]-pyridin-2-carbonsäure- (2-methoxyethyl)amid

### Beispiel 181

5-[((3-((3-Methoxypropyl)aminocarbonyl)phenylsulfonyl)amino)carbonyl]-pyridin-2-carbonsäure-(2-methoxyethyl)amid

### Beispiel 182

5-[((3-((2-Phenylethyl)aminocarbonyl)phenylsulfonyl)amino)carbonyl]-pyridin-2-carbonsäure-(2-methoxymethyl)amid

### Beispiel 183

5-[((3-((2-Phenoxyethyl)aminocarbonyl)phenylsulfonyl)amino)carbonyl]-pyridin-2-carbonsäure-(2-methoxymethyl)amid

### Beispiel 184

5-[((4-(Benzylaminocarbonyl)phenylsulfonyl)amino)carbonyl]-pyridin-2-carbonsäure-(2-methoxyethyl)amid

### Beispiel 185

5-[((4-((3-Phenylpropyl)aminocarbonyl)phenylsulfonyl)amino)carbonyl]-pyridin-2-carbonsäure-(2-ethoxyethyl)amid

### Beispiel 186

5-[((4-((4-Phenyl-n-butyl)aminocarbonyl)phenylsulfonyl)amino)carbonyl]-pyridin-2-carbonsäure- (2-ethoxyethyl)amid

### Beispiel 187

5-[((4-((2-(4-Methoxyphenyl)ethyl)aminocarbonyl)phenylsulfonyl)amino)carbonyl]-pyridin-2-carbonsäure-(2-ethoxyethyl)amid

### Beispiel 188

5-[((4-((2-(3,4-Dimethoxyphenyl)ethyl)aminocarbonyl)phenylsulfonyl)amino) carbonyl]-pyridin-2-carbonsäure-(2-ethoxyethyl)amid

### Beispiel 189

5-[((4-((2-(2-Methoxyphenyl)ethyl)aminocarbonyl)phenylsulfonyl)amino)carbonyl]-pyridin-2-carbonsäure-(2-ethoxyethyl)amid

### Beispiel 190

5-[((4-((2-(4-Fluorphenyl)ethyl)aminocarbonyl)phenylsulfonyl)amino)carbonyl]-pyridin-2-carbonsäure-2-(ethoxyethyl)amid

### Beispiel 191

5-[((4-((2-(4-Chlorphenyl)ethyl)aminocarbonyl)phenylsulfonyl)amino)carbonyl]-pyridin-2-carbonsäure-(2-ethoxyethyl)amid

### Beispiel 192

5-[((4-(Ethylaminocarbonyl)phenylsulfonyl)amino)carbonyl]-pyridin-2-carbonsäure-(2-ethoxyethyl)amid

### Beispiel 193

5-[((4-(n-Butylaminocarbonyl)phenylsulfonyl)amino)carbonyl]-pyridin-2-carbonsäure-(2-ethoxyethyl)amid

### Beispiel 194

5-[((4-(n-Hexylaminocarbonyl)phenylsulfonyl)amino)carbonyl]-pyridin-2-carbonsäure-(2-ethoxyethyl)amid

### Beispiel 195

5-[((4-(N,N-Di-n-butylaminocarbonyl)phenylsulfonyl)amino)carbonyl]-pyridin-2-carbonsäure- (2-ethoxyethyl)amid

### Beispiel 196

5-[((4-(Cyclohexylaminocarbonyl)phenylsulfonyl)amino)carbonyl]-pyridin-2-carbonsäure-(2-ethoxyethyl)amid

### Beispiel 197

5-[((4-((2-Methoxyethyl)aminocarbonyl)phenylsulfonyl)amino)carbonyl]-pyridin-2-carbonsäure- (2-ethoxyethyl)amid

### Beispiel 198

5-[((4-((2-Ethoxyethyl)aminocarbonyl)phenylsulfonyl)amino)carbonyl]-pyridin-2-carbonsäure-(2-ethoxyethyl)amid

### Beispiel 199

5-[((4-((3-Ethoxypropyl)aminocarbonyl)phenylsulfonyl)amino)carbonyl]-pyridin-2-carbonsäure-(2-ethoxyethyl)amid

### Beispiel 200

5-[((4-((2-Phenylethyl)aminocarbonyl)phenylsulfonyl)amino)carbonyl]-pyridin-2-carbonsäure-2(ethoxyethyl)amid

### Beispiel 201

5-[((4-((2-Phenoxyethyl)aminocarbonyl)phenylsulfonyl)amino)carbonyl]-pyridin-2-carbonsäure-(2-ethoxyethyl)amid

### Beispiel 202

5-[((4-((2-Phenylethyl)aminocarbonyl)phenylsulfonyl)amino)carbonyl]-pyridin-2-carbonsäure-(glycylmethylester)amid-Natriumsalz
a) 5-[((4-((2-Phenylethyl)aminocarbonyl)phenylsulfonyl)amino)carbonyl]-pyridin-2-carbonsäure
   0,7 g (1,5 mmol) des in Beispiel 112 b) beschriebenen Methylesters wurden bei 20° C unter Rühren in 100 ml 1,5 n methanolische NaOH eingetragen. Nachdem eine Lösung entstanden war, fällt später ein kristallines Produkt aus. Dann wurde noch 30 min gerührt. Man engte im Vakuum ein, löste den Rückstand in einem Gemisch aus Wasser und Tetrahydrofuran, säuerte mit wäßriger HCI auf pH = 1 an, engte im Vakuum ein und saugte das farblos kristalline Produkt ab. Man erhielt 0,6 g, Fp. 263° C (u. Zers.).
b) 2,3 g (5,07 mmol) des vorstehenden Pyridin-2-carbonsäure-Derivats wurden in 200 ml wasserfreiem Acetonitril/Tetrahydrofuran-Gemisch suspendiert und nacheinander mit 0,7 g (5,5 mmol) Glycinmethylester-Hydrochlorid, 1,4 ml (11 mmol) N-Ethyl-morpholin, 0,73 (5,5 mmol) 1-Hydroxy-benzotriazol und 1,14 g (5,5 mmol) N,N'-Dicyclohexylcarbodiimid versetzt und 20 h bei 20° C gerührt. Dann wurde vom Ungelösten abfiltriert, das Filtrat im Vakuum eingeengt, in 200 ml Dichlormethan aufgenommen und zweimal mit gesättigter wäßriger NaHCO₃-Lösung extrahiert. Aus der NaHCO₃-Phase kristallisierten 0,75 g Produkt, Fp. 275-277° C. Aus der organischen Phase erhielt man nach Trocknen, Einengen und Behandeln des Rückstandes mit Ethylacetat weitere 0,42 g farblos kristallinen Produkts, Fp. 275-277° C (unter Schäumen).

### Beispiel 203

5-[((4-(Benzylaminocarbonyl)phenylsulfonyl)amino)carbonyl]-pyridin-2-carbonsäure-(glycylmethylester)amid

### Beispiel 204

5-[((4-((3-Phenylpropyl)aminocarbonyl)phenylsulfonyl)amino)carbonyl]-pyridin-2-carbonsäure-(glycylmethylester)amid

### Beispiel 205

5-[((4-((4-Phenyl-n-butyl)aminocarbonyl)phenylsulfonyl)amino)carbonyl]-pyridin-2-carbonsäure-(glycylmethylester)amid

### Beispiel 206

5-[((4-((2-(4-Methoxyphenyl)ethyl)aminocarbonyl)phenylsulfonyl)amino)carbonyl]-pyridin-2-carbonsäure-(glycylmethylester)amid

### Beispiel 207

5-[((4-((2-(3,4-Dimethoxyphenyl)ethyl)aminocarbonyl)phenylsulfonyl)amino) carbonyl]-pyridin-2-carbonsäure-(glycylmethylester)amid-Natriumsalz

### Fp. 190-191° C (Methanol)

### Beispiel 208

5-[((4-((2-(2-Methoxyphenyl)ethyl)aminocarbonyl)phenylsulfonyl)amino)carbonyl]-pyridin-2-carbonsäure-(glycylmethylester)amid

### Beispiel 209

5-[((4-((2-(4-Fluorphenyl)ethyl)aminocarbonyl)phenylsulfonyl)amino)carbonyl]-pyridin-2-carbonsäure-2-(glycylmethylester)amid

### Beispiel 210

5-[((4-((2-(4-Chlorphenyl)ethyl)aminocarbonyl)phenylsulfonyl)amino)carbonyl]-pyridin-2-carbonsäure-(glycylmethylester)amid

### Beispiel 211

5-[((4-(Ethylaminocarbonyl)phenylsulfonyl)amino)carbonyl]-pyridin-2-carbonsäure-(glycylmethylester)amid

### Beispiel 212

5-[((4-(n-Butylaminocarbonyl)phenylsulfonyl)amino)carbonyl]-pyridin-2-carbonsäure-(glycylmethylester)amid
Fp. 215-217° C (Methanol)

### Beispiel 213

5-[((4-(n-Hexylaminocarbonyl)phenylsulfonyl)amino)carbonyl]-pyridin-2-carbonsäure-(glycylmethylester)amid

### Beispiel 214

5-[((4-(N,N-Di-n-butylaminocarbonyl)phenylsulfonyl)amino)carbonyl]-pyridin-2-carbonsäure-(glycylmethylester)amid

### Beispiel 215

5-[((4-(Cyclohexylaminocarbonyl)phenylsulfonyl)amino)carbonyl]-pyridin-2-carbonsäure-(glycylmethylester)amid

### Beispiel 216

5-[((4-((2-Methoxyethyl)aminocarbonyl)phenylsulfonyl)amino)carbonyl]-pyridin-2-carbonsäure-(glycylmethylester)amid

### Beispiel 217

5-[((4-((2-Ethoxyethyl)aminocarbonyl)phenylsulfonyl)amino)carbonyl]-pyridin-2-carbonsäure-(glycylmethylester)amid

### Beispiel 218

5-[((4-((3-Ethoxypropyl)aminocarbonyl)phenylsulfonyl)amino)carbonyl]-pyridin-2-carbonsäure-(glycylmethylester)amid
Fp. 171-173° C (Methanol)

### Beispiel 219

5-[((4-((2-Phenoxyethyl)aminocarbonyl)phenylsulfonyl)amino)carbonyl]-pyridin-2-carbonsäure-(glycylmethylester)amid

### Beispiel 220

5-[((3-(Benzylaminocarbonyl)phenylsulfonyl)amino)carbonyl]-pyridin-2-carbonsäure-(glycylmethylester)amid

### Beispiel 221

5-[((3-((3-Phenylpropyl)aminocarbonyl)phenylsulfonyl)amino)carbonyl]-pyridin-2-carbonsäure-(glycylmethylester)amid

### Beispiel 222

5-[((3-((4-Phenyl-n-butyl)aminocarbonyl)phenylsulfonyl)amino)carbonyl]-pyridin-2-carbonsäure-(glycylmethylester)amid

### Beispiel 223

5-[((3-((2-(4-Methoxyphenyl)ethyl)aminocarbonyl)phenylsulfonyl)amino)carbonyl]-pyridin-2-carbonsäure-(glycylmethylester)amid

### Beispiel 224

5-[((3-((2-(3-Methoxyphenyl)ethyl)aminocarbonyl)phenylsulfonyl)amino)carbonyl]-pyridin-2-carbonsäure-(glycylmethylester)amid

### Beispiel 225

5-[((3-((2-(2-Methoxyphenyl)ethyl)aminocarbonyl)phenylsulfonyl)amino)carbonyl]-pyridin-2-carbonsäure-(glycylmethylester)amid

### Beispiel 226

5-[((3-((2-(4-Fluorphenyl)ethyl)aminocarbonyl)phenylsulfonyl)amino)carbonyl]-pyridin-2-carbonsäure-2-(glycylmethylester)amid

### Beispiel 227

5-[((3-((2-(4-Chlorphenyl)ethyl)aminocarbonyl)phenylsulfonyl)amino)carbonyl]-pyridin-2-carbonsäure-(glycylmethylester)amid

### Beispiel 228

5-[((3-(Ethylaminocarbonyl)phenylsulfonyl)amino)carbonyl]-pyridin-2-carbonsäure-(glycylmethylester)amid

### Beispiel 229

5-[((3-(n-Butylaminocarbonyl)phenylsulfonyl)amino)carbonyl]-pyridin-2-carbonsäure-(glycylmethylester)amid
Fp. 198-200° C (Methanol)

### Beispiel 230

5-[((3-(n-Hexylaminocarbonyl)phenylsulfonyl)amino)carbonyl]-pyridin-2-carbonsäure-(glycylmethylester)amid

### Beispiel 231

5-[((3-(N,N-Di-n-butylaminocarbonyl)phenylsulfonyl)amino)carbonyl]-pyridin-2-carbonsäure-(glycylmethylester)amid

### Beispiel 232

5-[((3-(Cyclohexylaminocarbonyl)phenylsulfonyl)amino)carbonyl]-pyridin-2-carbonsäure-(glycylmethylester)amid

### Beispiel 233

5-[((3-((2-Methoxyethyl)aminocarbonyl)phenylsulfonyl)amino)carbonyl]-pyridin-2-carbonsäure-(glycylmethylester)amid

### Beispiel 234

5-[((3-((2-Ethoxyethyl)aminocarbonyl)phenylsulfonyl)amino)carbonyl]-pyridin-2-carbonsäure-(glycylmethylester)amid

### Beispiel 235

5-[((3-((3-Ethoxypropyl)aminocarbonyl)phenylsulfonyl)amino)carbonyl]-pyridin-2-carbonsäure-(glycylmethylester)amid

### Beispiel 236

5-[((3-((2-Phenylethyl)aminocarbonyl)phenylsulfonyl)amino)carbonyl]-pyridin-2-carbonsäure-(glycylmethylester)amid

### Beispiel 237

5-[((3-((2-Phenoxyethyl)aminocarbonyl)phenylsulfonyl)amino)carbonyl]-pyridin-2-carbonsäure-(glycylmethylester)amid

### Beispiel 238

5-[((4-(Benzylaminocarbonyl)phenylsulfonyl)amino)carbonyl]-pyridin-2-carbonsäure-(glycylethylester)amid

### Beispiel 239

5-[((4-((3-Phenylpropyl)aminocarbonyl)phenylsulfonyl)amino)carbonyl]-pyridin-2-carbonsäure-(glycylethylester)amid

### Beispiel 240

5-[((4-((4-Phenyl-n-butyl)aminocarbonyl)phenylsulfonyl)amino)carbonyl]-pyridin-2-carbonsäure-(glycylethylester)amid

### Beispiel 241

5-[((4-((2-(4-Methoxyphenyl)ethyl)aminocarbonyl)phenylsulfonyl)amino)carbonyl]-pyridin-2-carbonsäure-(glycylethylester)amid

### Beispiel 242

5-[((4-((2-(3,4-Dimethoxyphenyl)ethyl)aminocarbonyl)phenylsulfonyl)amino) carbonyl]-pyridin-2-carbonsäure-(glycylethylester)amid

### Beispiel 243

5-[((4-((2-(2-Methoxyphenyl)ethyl)aminocarbonyl)phenylsulfonyl)amino)carbonyl]-pyridin-2-carbonsäure-(glycylethylester)amid

### Beispiel 244

5-[((4-((2-(4-Fluorphenyl)ethyl)aminocarbonyl)phenylsulfonyl)amino)carbonyl]-pyridin-2-carbonsäure-2-(glycylethylester)amid

### Beispiel 245

5-[((4-((2-(4-Chlorphenyl)ethyl)aminocarbonyl)phenylsulfonyl)amino)carbonyl]-pyridin-2-carbonsäure-(glycylethylester)amid

### Beispiel 246

5-[((4-(Ethylaminocarbonyl)phenylsulfonyl)amino)carbonyl]-pyridin-2-carbonsäure-(glycylethylester)amid

### Beispiel 247

5-[((4-(n-Butylaminocarbonyl)phenylsulfonyl)amino)carbonyl]-pyridin-2-carbonsäure-(glycylethylester)amid

### Beispiel 248

5-[((4-(n-Hexylaminocarbonyl)phenylsulfonyl)amino)carbonyl]-pyridin-2-carbonsäure-(glycylethylester)amid

### Beispiel 249

5-[((4-(N,N-Di-n-butylaminocarbonyl)phenylsulfonyl)amino)carbonyl]-pyridin-2-carbonsäure-(glycylethylester)amid

### Beispiel 250

5-[((4-(Cyclohexylaminocarbonyl)phenylsulfonyl)amino)carbonyl]-pyridin-2-carbonsäure-(glycylethylester)amid

### Beispiel 251

5-[((4-((2-Methoxyethyl)aminocarbonyl)phenylsulfonyl)amino)carbonyl]-pyridin-2-carbonsäure-(glycylethylester)amid

### Beispiel 252

5-[((4-((2-Ethoxyethyl)aminocarbonyl)phenylsulfonyl)amino)carbonyl]-pyridin-2-carbonsäure-(glycylethylester)amid

### Beispiel 253

5-[((4-((3-Ethoxypropyl)aminocarbonyl)phenylsulfonyl)amino)carbonyl]-pyridin-2-carbonsäure-(glycylethylester)amid

### Beispiel 254

5-[((4-((2-Phenylethyl)aminocarbonyl)phenylsulfonyl)amino)carbonyl]-pyridin-2-carbonsäure-(glycylethylester)amid

### Beispiel 255

5-[((4-((2-Phenoxyethyl)aminocarbonyl)phenylsulfonyl)amino)carbonyl]-pyridin-2-carbonsäure-(glycylethylester)amid

### Beispiel 256

5-[((4-(Benzylaminocarbonyl)phenylsulfonyl)amino)carbonyl]-pyridin-2-carbonsäure-(glycyl-2-propylester)amid

### Beispiel 257

5-[((4-((3-Phenylpropyl)aminocarbonyl)phenylsulfonyl)amino)carbonyl]-pyridin-2-carbonsäure-(glycyl-2-propylester)amid

### Beispiel 258

5-[((4-((4-Phenyl-n-butyl)aminocarbonyl)phenylsulfonyl)amino)carbonyl]-pyridin-2-carbonsäure-(glycyl-2-propylester)amid

### Beispiel 259

5-[((4-((2-(4-Methoxyphenyl)ethyl)aminocarbonyl)phenylsulfonyl)amino)carbonyl]-pyridin-2-carbonsäure-(glycyl-2-propylester)amid

### Beispiel 260

5-[((4-((2-(3,4-Dimethoxyphenyl)ethyl)aminocarbonyl)phenylsulfonyl)amino) carbonyl]-pyridin-2-carbonsäure-(glycyl-2-propylester)amid

### Beispiel 261

5-[((4-((2-(2-Methoxyphenyl)ethyl)aminocarbonyl)phenylsulfonyl)amino)carbonyl]-pyridin-2-carbonsäure-(glycyl-2-propylester)amid

### Beispiel 262

5-[((4-((2-(4-Fluorphenyl)ethyl)aminocarbonyl)phenylsulfonyl)amino)carbonyl]-pyridin-2-carbonsäure-2-(glycyl-2-propylester)amid

### Beispiel 263

5-[((4-((2-(4-Chlorphenyl)ethyl)aminocarbonyl)phenylsulfonyl)amino)carbonyl]-pyridin-2-carbonsäure-(glycyl-2-propylester)amid

### Beispiel 264

5-[((4-(Ethylaminocarbonyl)phenylsulfonyl)amino)carbonyl]-pyridin-2-carbonsäure-(glycyl-2-propylester)amid

### Beispiel 265

5-[((4-(n-Butylaminocarbonyl)phenylsulfonyl)amino)carbonyl]-pyridin-2-carbonsäure-(glycyl-2-propylester)amid

### Beispiel 266

5-[((4-(n-Hexylaminocarbonyl)phenylsulfonyl)amino)carbonyl]-pyridin-2-carbonsäure-(glycyl-2-propylester)amid

### Beispiel 267

5-[((4-(N,N-Di-n-butylaminocarbonyl)phenylsulfonyl)amino)carbonyl]-pyridin-2-carbonsäure-(glycyl-2-propylester)amid

### Beispiel 268

5-[((4-(Cyclohexylaminocarbonyl)phenylsulfonyl)amino)carbonyl]-pyridin-2-carbonsäure-(glycyl-2-propylester)amid

### Beispiel 269

5-[((4-((2-Methoxyethyl)aminocarbonyl)phenylsulfonyl)amino)carbonyl]-pyridin-2-carbonsäure-(glycyl-2-propylester)amid

### Beispiel 270

5-[((4-((2-Ethoxyethyl)aminocarbonyl)phenylsulfonyl)amino)carbonyl]-pyridin-2-carbonsäure-(glycyl-2-propylester)amid

### Beispiel 271

5-[((4-((3-Ethoxypropyl)aminocarbonyl)phenylsulfonyl)amino)carbonyl]-pyridin-2-carbonsäure-(glycyl-2-propylester)amid

### Beispiel 272

5-[((4-((2-Phenylethyl)aminocarbonyl)phenylsulfonyl)amino)carbonyl]-pyridin-2-carbonsäure-(glycyl-2-propylester)amid

### Beispiel 273

5-[((4-((2-Phenoxyethyl)aminocarbonyl)phenylsulfonyl)amino)carbonyl]-pyridin-2-carbonsäure-(glycyl-2-propylester)amid

### Beispiel 274

5-[((4-(Benzylaminocarbonyl)phenylsulfonyl)amino)carbonyl]-pyridin-2-carbonsäure-(glycylbenzylester)amid

### Beispiel 275

5-[((4-((3-Phenylpropyl)aminocarbonyl)phenylsulfonyl)amino)carbonyl]-pyridin-2-carbonsäure-(glycylbenzylester)amid

### Beispiel 276

5-[((4-((4-Phenyl-n-butyl)aminocarbonyl)phenylsulfonyl)amino)carbonyl]-pyridin-2-carbonsäure-(glycylbenzylester)amid

### Beispiel 277

5-[((4-((2-(4-Methoxyphenyl)ethyl)aminocarbonyl)phenylsulfonyl)amino)carbonyl]-pyridin-2-carbonsäure-(glycylbenzylester)amid

### Beispiel 278

5-[((4-((2-(3,4-Dimethoxyphenyl)ethyl)aminocarbonyl)phenylsulfonyl)amino) carbonyl]-pyridin-2-carbonsäure-(glycylbenzylester)amid

### Beispiel 279

5-[((4-((2-(2-Methoxyphenyl)ethyl)aminocarbonyl)phenylsulfonyl)amino)carbonyl]-pyridin-2-carbonsäure-(glycylbenzylester)amid

### Beispiel 280

5-[((4-((2-(4-Fluorphenyl)ethyl)aminocarbonyl)phenylsulfonyl)amino)carbonyl]-pyridin-2-carbonsäure-2-(glycylbenzylester)amid

### Beispiel 281

5-[((4-((2-(4-Chlorphenyl)ethyl)aminocarbonyl)phenylsulfonyl)amino)carbonyl]-pyridin-2-carbonsäure-(glycylbenzylester)amid

### Beispiel 282

5-[((4-(Ethylaminocarbonyl)phenylsulfonyl)amino)carbonyl]-pyridin-2-carbonsäure-(glycylbenzylester)amid

### Beispiel 283

5-[((4-(n-Butylaminocarbonyl)phenylsulfonyl)amino)carbonyl]-pyridin-2-carbonsäure-(glycylbenzylester)amid

### Beispiel 284

5-[((4-(n-Hexylaminocarbonyl)phenylsulfonyl)amino)carbonyl]-pyridin-2-carbonsäure-(glycylbenzylester)amid

### Beispiel 285

5- [((4-(N,N-Di-n-butylaminocarbonyl)phenylsulfonyl)amino)carbonyl]-pyridin-2-carbonsäure-(glycylbenzylester)amid

### Beispiel 286

5-[((4-(Cyclohexylaminocarbonyl)phenylsulfonyl)amino)carbonyl]-pyridin-2-carbonsäure-(glycylbenzylester)amid

### Beispiel 287

5-[((4-((2-Methoxyethyl)aminocarbonyl)phenylsulfonyl)amino)carbonyl]-pyridin-2-carbonsäure-(glycylbenzylester)amid

### Beispiel 288

5-[((4-((2-Ethoxyethyl)aminocarbonyl)phenylsulfonyl)amino)carbonyl]-pyridin-2-carbonsäure-(glycylbenzylester)amid

### Beispiel 289

5-[((4-((3-Ethoxypropyl)aminocarbonyl)phenylsulfonyl)amino)carbonyl]-pyridin-2-carbonsäure-(glycylbenzylester)amid

### Beispiel 290

5-[((4-((2-Phenylethyl)aminocarbonyl)phenylsulfonyl)amino)carbonyl]-pyridin-2-carbonsäure-(glycylbenzylester)amid

### Beispiel 291

5-[((4-((2-Phenoxyethyl)aminocarbonyl)phenylsulfonyl)amino)carbonyl]-pyridin-2-carbonsäure-(glycylbenzylester)amid

### Beispiel 292

5-[((3-(n-Butylaminocarbonyl)phenylsulfonyl)amino)carbonyl]-pyridin-2-carbonsäure-glycylamid
Fp. 220-220° C (aus wäßriger Salzsäure)

### Beispiel 293

5-[((4-((3-Phenylpropyl)aminocarbonyl)phenylsulfonyl)amino)carbonyl]-pyridin-2-carbonsäure-glycylamid

### Beispiel 294

5-[((4-((4-Phenyl-n-butyl)aminocarbonyl)phenylsulfonyl)amino)carbonyl]-pyridin-2-carbonsäure-glycylamid

### Beispiel 295

5-[((4-((2-(4-Methoxyphenyl)ethyl)aminocarbonyl)phenylsulfonyl)amino)carbonyl]-pyridin-2-carbonsäure-glycylamid

### Beispiel 296

5-[((4-((2-(3,4-Dimethoxyphenyl)ethyl)aminocarbonyl)phenylsulfonyl)amino) carbonyl]-pyridin-2-carbonsäure-glycylamid
Fp. 190° C (Sintern), 201-203° C (aus wäßriger Salzsäure)

### Beispiel 297

5-[((4-((2-(2-Methoxyphenyl)ethyl)aminocarbonyl)phenylsulfonyl)amino)carbonyl]-pyridin-2-carbonsäure-glycylamid

### Beispiel 298

5-[((4-((2-(4-Fluorphenyl)ethyl)aminocarbonyl)phenylsulfonyl)amino)carbonyl]-pyridin-2-carbonsäure-2-glycylamid

### Beispiel 299

5-[((4-((2-(4-Chlorphenyl)ethyl)aminocarbonyl)phenylsulfonyl)amino)carbonyl]-pyridin-2-carbonsäure-glycylamid

### Beispiel 300

5-[((4-(Ethylaminocarbonyl)phenylsulfonyl)amino)carbonyl]-pyridin-2-carbonsäure-glycylamid

### Beispiel 301

5-[((4-(n-Butylaminocarbonyl)phenylsulfonyl)amino)carbonyl]-pyridin-2-carbonsäure-glycylamid
Fp. 244-247° C (Zersetzung, aus wäßriger Salzsäure)

### Beispiel 302

5-[((4-(n-Hexylaminocarbonyl)phenylsulfonyl)amino)carbonyl]-pyridin-2-carbonsäure-glycylamid

### Beispiel 303

5-[((4-(N,N-Di-n-butylaminocarbonyl)phenylsulfonyl)amino)carbonyl]-pyridin-2-carbonsäure-glycylamid

### Beispiel 304

5-[((4-(Cyclohexylaminocarbonyl)phenylsulfonyl)amino)carbonyl]-pyridin-2-carbonsäure-glycylamid

### Beispiel 305

5-[((4-((2-Methoxyethyl)aminocarbonyl)phenylsulfonyl)amino)carbonyl]-pyridin-2-carbonsäure-glycylamid

### Beispiel 306

5-[((4-((2-Ethoxyethyl)aminocarbonyl)phenylsulfonyl)amino)carbonyl]-pyridin-2-carbonsäure-glycylamid

### Beispiel 307

5-[((4-((3-Ethoxypropyl)aminocarbonyl)phenylsulfonyl)amino)carbonyl]-pyridin-2-carbonsäure-glycylamid
Fp. 190° C (aus Tetrahydrofuran/wäßriger Salzsäure)

### Beispiel 308

5-[((4-((2-Phenylethyl)aminocarbonyl)phenylsulfonyl)amino)carbonyl]-pyridin-2-carbonsäure-glycylamid
Fp. 263-265° C (aus wäßriger Salzsäure)

### Beispiel 309

5-[((4-((2-Phenoxyethyl)aminocarbonyl)phenylsulfonyl)amino)carbonyl]-pyridin-2-carbonsäure-glycylamid

### Beispiel 310

5-[((2-Chlor-4-((2-phenoxyethyl)aminocarbonyl)phenylsulfonyl)amino)carbonyl]-pyridin-2-carbonsäure-(2-hydroxyethyl)amid

### Beispiel 311

5-[((2-Chlor-4-((3-ethoxypropyl)aminocarbonyl)phenylsulfonyl)amino)carbonyl]-pyridin-2-carbonsäure-(2-hydroxyethyl)amid

### Beispiel 312

5-[((4-Chlor-3-((3-ethoxypropyl)aminocarbonyl)phenylsulfonyl)amino)carbonyl]-pyridin-2-carbonsäure-(2-hydroxyethyl)amid

### Beispiel 313

5-[((4-Chlor-3-((2-phenoxyethyl)aminocarbonyl)phenylsulfonyl)amino)carbonyl]-pyridin-2-carbonsäure-(2-hydroxyethyl)amid

### Beispiel 314

5-[((2-Chlor-4-((2-phenoxyethyl)aminocarbonyl)phenylsulfonyl)amino)carbonyl]-pyridin-2-carbonsäure-(2-methoxyethyl)amid

### Beispiel 315

5-[((2-Chlor-4-((3-ethoxypropyl)aminocarbonyl)phenylsulfonyl)amino)carbonyl]-pyridin-2-carbonsäure-(2-methoxyethyl)amid

### Beispiel 316

5-[((4-Chlor-3-((3-ethoxypropyl)aminocarbonyl)phenylsulfonyl)amino)carbonyl]-pyridin-2-carbonsäure-(2-methoxyethyl)amid

### Beispiel 317

5-[((4-Chlor-3-((2-phenoxyethyl)aminocarbonyl)phenylsulfonyl)amino)carbonyl]-pyridin-2-carbonsäure-(2-methoxyethyl)amid

### Beispiel 318

5-[((2-Chlor-4-((2-phenoxyethyl)aminocarbonyl)phenylsulfonyl)amino)carbonyl]-pyridin-2-carbonsäure-(2-ethoxyethyl)amid

### Beispiel 319

5-[((2-Chlor-4-((3-ethoxypropyl)aminocarbonyl)phenylsulfonyl)amino)carbonyl]-pyridin-2-carbonsäure-(ethoxyethyl)amid

### Beispiel 320

5-[((4-Chlor-3-((3-ethoxypropyl)aminocarbonyl)phenylsulfonyl)amino)carbonyl]-pyridin-2-carbonsäure-(2-ethoxyethyl)amid

### Beispiel 321

5-[((4-Chlor-3-((2-phenoxyethyl)aminocarbonyl)phenylsulfonyl)amino)carbonyl]-pyridin-2-carbonsäure-(2-ethoxyethyl)amid

### Beispiel 322

5-[((2-Chlor-4-((2-phenoxyethyl)aminocarbonyl)phenylsulfonyl)amino)carbonyl]-pyridin-2-carbonsäure-(2-(2-propyl)oxyethyl)amid

### Beispiel 323

5-[((2-Chlor-4-((3-ethoxypropyl)aminocarbonyl)phenylsulfonyl)amino)carbonyl]-pyridin-2-carbonsäure-(2-(2-propyl)oxyethyl)amid

### Beispiel 324

5-[((2-Chlor-4-((2-phenoxyethyl)aminocarbonyl)phenylsulfonyl)amino)carbonyl]-pyridin-2-carbonsäure-(glycylmethylester)amid

### Beispiel 325

5-[((2-Chlor-4-((3-ethoxypropyl)aminocarbonyl)phenylsulfonyl)amino)carbonyl]-pyridin-2-carbonsäure-(glycylmethylester)amid

### Beispiel 326

5-[((4-Chlor-3-((3-ethoxypropyl)aminocarbonyl)phenylsulfonyl)amino)carbonyl]-pyridin-2-carbonsäure-(glycylmethylester)amid

### Beispiel 327

5-[((4-Chlor-3-((2-phenoxyethyl)aminocarbonyl)phenylsulfonyl)amino)carbonyl]-pyridin-2-carbonsäure-(glycylmethylester)amid

### Beispiel 328

5-[((2-Chlor-4-((2-phenoxyethyl)aminocarbonyl)phenylsulfonyl)amino)carbonyl]-pyridin-2-carbonsäure-(glycyl-2-propylester)amid

### Beispiel 329

5-[((2-Chlor-4-((3-ethoxypropyl)aminocarbonyl)phenylsulfonyl)amino)carbonyl]-pyridin-2-carbonsäure-(glycyl-2-propylester)amid

### Beispiel 330

5-[((2-Chlor-4-((2-phenoxyethyl)aminocarbonyl)phenylsulfonyl)amino)carbonyl]-pyridin-2-carbonsäure-(glycyl-3-pentylester)amid

### Beispiel 331

5-[((2-Chlor-4-((3-ethoxypropyl)aminocarbonyl)phenylsulfonyl)amino)carbonyl]-pyridin-2-carbonsäure-(glycyl-3-pentylester)amid

### Beispiel 332

5-[((4-(2-((2-Chlor-5-methoxybenzoyl)amino)ethyl)phenylsulfonyl)amino)carbonyl]-pyridin-2-carbonsäure-(2-hydroxyethyl)amid
a) 5-[((4-(2-((2-Chlor-5-methoxybenzoyl)amino)ethyl)phenylsulfonyl)amino)carbonyl]-pyridin-2-carbonsäure-methylester
   Das aus 4,0 g (22 mmol) Pyridin-2,5-dicarbonsäure-2-methylester, wie in Beispiel 1 c) beschrieben, hergestellte Pyridin-2-carbonsäuremethylester-5-carbonsäurechlorid wurde in 50 ml wasserfreiem 1,4-Dioxan bei 40° C zu dem Reaktionsgemisch aus 7,4 g (20 mmol) 2-(((2-Chlor-5-methoxy-benzoyl)amino)ethyl) benzolsulfonsäureamid (hergestellt aus 4-(2-Aminoethyl)benzolsulfonsäureamid und 2-Chlor-5-methoxy-benzoesäure), 2,3 g (20 mmol) Kalium-tert.butylat in 150 ml wasserfreiem 1,4-Dioxan hinzugefügt (zur Bildung des Sulfonamid-Natriumsalzes war 15 min bei 50° C gerührt worden).
   Die Reaktionsmischung wurde 90 min bei 60° C, dann 2 h unter Rückfluß gerührt, das Lösungsmittel im Vakuum abdestilliert, der Rückstand mit Wasser versetzt, mit wäßriger HCI auf pH 1-2 gebracht und mit Dichlormethan extrahiert.
   Der Rückstand wurde mit heißem Ethylacetat behandelt, abgesaugt und mit Ethylacetat gewaschen. Das so erhaltene Rohprodukt (2,8 g) wurde mit 100 ml kaltem Wasser, dann mit 100 ml heißem Wasser behandelt und das farblos kristalline Produkt abgesaugt. Man erhielt 2,6 g, Fp. 187-190° C.
b) 0,85 g (1,6 mmol) des vorstehenden Methylesters wurden in 150 ml 2-Aminoethanol 1 h bei 80 bis 90° C gerührt. Nach dem Abkühlen wurde überschüssiges Reagenz im Vakuum abdestilliert, der Rückstand in 20-30 ml Wasser gelöst, mit konz. wäßriger HCI auf pH 1 gebracht, des kristalline Produkt abgesaugt, mit Wasser gewaschen, mit heißem Ethylacetat behandelt und wiederum abgesaugt. Man erhielt 0,65 g der Titelverbindung, Fp. 135-137° C.

### Beispiel 333

5-[((4-(2-(Acetylamino)ethyl)phenylsulfonyl)amino)carbonyl]-pyridin-2-carbonsäure-(2-hydroxyethyl)amid

### Beispiel 334

5-[((4-(2-(n-Butanoylamino)ethyl)phenylsulfonyl)amino)carbonyl]-pyridin-2-carbonsäure-(2-hydroxyethyl)amid

### Beispiel 335

5-[((4-(2-(Benzoylamino)ethyl)phenylsulfonyl)amino)carbonyl]-pyridin-2-carbonsäure-(2-hydroxyethyl)amid

### Beispiel 336

5-[((4-(2-((4-Chlorbenzoyl)amino)ethyl)phenylsulfonyl)amino)carbonyl]-pyridin-2-carbonsäure-(2-hydroxyethyl)amid

### Beispiel 337

5-[((4-(2-((5-Chlor-2-methoxybenzoyl)amino)ethyl)phenylsulfonyl)amino)carbonyl]-pyridin-2-carbonsäure-(2-hydroxyethyl)amid

### Beispiel 338

5-[((4-(2-((3-3,4-Dimethoxyphenylpropionyl)amino)ethyl)phenylsulfonyl)amino)carbonyl]-pyridin-2-carbonsäure-(2-hydroxyethyl)amid
Fp. 176-177° C (Wasser)

### Beispiel 339

5-[((4-(2-((2-Phenylacetyl)amino)ethyl)phenylsulfonyl)amino)carbonyl]-pyridin-2-carbonsäure-(2-hydroxyethyl)amid

### Beispiel 340

5-[((4-(2-((Phenoxyacetyl)amino)ethyl)phenylsulfonyl)amino)carbonyl]-pyridin-2-carbonsäure-(2-hydroxyethyl)amid
Fp. 155-156° C (Wasser)

### Beispiel 341

5-[((4-(2-((4-Fluorbenzoyl)amino)ethyl)phenylsulfonyl)amino)carbonyl]-pyridin-2-carbonsäure-(2-hydroxyethyl)amid

### Beispiel 342

5-[((4-(2-((4-Methylpentanoyl)amino)ethyl)phenylsulfonyl)amino)carbonyl]-pyridin-2-carbonsäure-(2-hydroxyethyl)amid
Fp. 179-181° C (Wasser)

### Beispiel 343

5-[((4-(2-((Cyclohexanoyl)amino)ethyl)phenylsulfonyl)amino)carbonyl]-pyridin-2-carbonsäure-(2-hydroxyethyl)amid

### Beispiel 344

5-[((4-(2-((Cyclohexylacetyl)amino)ethyl)phenylsulfonyl)amino)carbhonyl]-pyridin-2-carbonsäure-(2-hydroxyethyl)amid

### Beispiel 345

5-[((4-(2-((2-Methylpropionyl)amino)ethyl)phenylsulfonyl)amino)carbonyl]-pyridin-2-carbonsäure-(2-hydroxyethyl)amid

### Beispiel 346

5-[((3-(2-((2-Chlor-5-methoxybenzoyl)amino)ethyl)phenylsulfonyl)amino)carbonyl]-pyridin-2-carbonsäure-(2-hydroxyethyl)amid

### Beispiel 347

5-[((3-(2-(Acetylamino)ethyl)phenylsulfonyl)amino)carbonyl]-pyridin-2-carbonsäure-(2-hydroxyethyl)amid

### Beispiel 348

5-[((3-(2-(n-Butanoylamino)ethyl)phenylsulfonyl)amino)carbonyl]-pyridin-2-carbonsäure-(2-hydroxyethyl)amid

### Beispiel 349

5-[((3-(2-(Benzoylamino)ethyl)phenylsulfonyl)amino)carbonyl]-pyridin-2-carbonsäure-(2-hydroxyethyl)amid

### Beispiel 350

5-[((3-(2-((4-Chlorbenzoyl)amino)ethyl)phenylsulfonyl)amino)carbonyl]-pyridin-2-carbonsäure-(2-hydroxyethyl)amid

### Beispiel 351

5-[((3-(2-((5-Chlor-2-methoxybenzoyl)amino)ethyl)phenylsulfonyl)amino)carbonyl]-pyridin-2-carbonsäure-(2-hydroxyethyl)amid

### Beispiel 352

5-[((3-(2-((3-Phenyl-n-propionyl)amino)ethyl)phenylsulfonyl)amino)carbonyl]-pyridin-2-carbonsäure-(2-hydroxyethyl)amid

### Beispiel 353

5-[((3-(2-((2-Phenylacetyl)amino)ethyl)phenylsulfonyl)amino)carbonyl]-pyridin-2-carbonsäure- (2-hydroxyethyl)amid

### Beispiel 354

5-[((3-(2-((Phenoxyacetyl)amino)ethyl)phenylsulfonyl)amino)carbonyl]-pyridin-2-carbonsäure-(2-hydroxyethyl)amid

### Beispiel 355

5-[((3-(2-((4-Fluorbenzoyl)amino)ethyl)phenylsulfonyl)amino)carbonyl]-pyridin-2-carbonsäure-(2-hydroxyethyl)amid

### Beispiel 356

5-[((3-(2-((4-Ethoxybenzoyl)amino)ethyl)phenylsulfonyl)amino)carbonyl]-pyridin-2-carbonsäure-(2-hydroxyethyl)amid

### Beispiel 357

5-[((3-(2-((Cyclohexanoyl)amino)ethyl)phenylsulfonyl)amino)carbonyl]-pyridin-2-carbonsäure-(2-hydroxyethyl)amid

### Beispiel 358

5-[((3-(2-((Cyclohexylacetyl)amino)ethyl)phenylsulfonyl)amino)carbhonyl]-pyridin-2-carbonsäure-(2-hydroxyethyl)amid

### Beispiel 359

5-[((3-(2-((2-Methylpropionyl)amino)ethyl)phenylsulfonyl)amino)carbonyl]-pyridin-2-carbonsäure-(2-hydroxyethyl)amid

### Beispiel 360

5-[((4-(2-((2-Chlor-5-methoxybenzoyl)amino)ethyl)phenylsulfonyl)amino)carbonyl]-pyridin-2-carbonsäure-(2-methoxyethyl)amid

### Beispiel 361

5-[((4-(2-(Acetylamino)ethyl)phenylsulfonyl)amino)carbonyl]-pyridin-2-carbonsäure-(2-methoxyethyl)amid

### Beispiel 362

5-[((4-(2-(n-Butanoylamino)ethyl)phenylsulfonyl)amino)carbonyl]-pyridin-2-carbonsäure-(2-methoxyethyl)amid

### Beispiel 363

5-[((4-(2-(Benzoylamino)ethyl)phenylsulfonyl)amino)carbonyl]-pyridin-2-carbonsäure-(2-methoxyethyl)amid

### Beispiel 364

5-[((4-(2-((4-Chlorbenzoyl)amino)ethyl)phenylsulfonyl)amino)carbonyl]-pyridin-2-carbonsäure-(2-methoxyethyl)amid

### Beipsiel 365

5-[((4-(2-((5-Chlor-2-methoxybenzoyl)amino)ethyl)phenylsulfonyl)amino)carbonyl]-pyridin-2-carbonsäure-(2-methoxyethyl)amid

### Beispiel 366

5-[((4-(2-((3-(3,4-Dimethoxyphenyl)-propionyl)amino)ethyl)phenylsulfonyl)amino) carbonyl]-pyridin-2-carbonsäure-(2-methoxyethyl)amid
Fp. 87-89° C (amorph, Diisopropylether)

### Beispiel 367

5-[((4-(2-((2-Phenylacetyl)amino)ethyl)phenylsulfonyl)amino)carbonyl]-pyridin-2-carbonsäure- (2-methoxyethyl)amid

### Beispiel 368

5-[((4-(2-((Phenoxyacetyl)amino)ethyl)phenylsulfonyl)amino)carbonyl]-pyridin-2-carbonsäure- (2-methoxyethyl)amid

### Beispiel 369

5-[((4-(2-((4-Fluorbenzoyl)amino)ethyl)phenylsulfonyl)amino)carbonyl]-pyridin-2-carbonsäure- (2-methoxyethyl)amid

### Beispiel 370

5-[((4-(2-((4-Methylpentanoyl)amino)ethyl)phenylsulfonyl)amino)carbonyl]-pyridin-2-carbonsäure-(2-methoxyethyl)amid
Fp. 154-157° C (Wasser)

### Beispiel 371

5-[((4-(2-((Cyclohexanoyl)amino)ethyl)phenylsulfonyl)amino)carbonyl]-pyridin-2-carbonsäure-(2-methoxyethyl)amid

### Beispiel 372

5-[((4-(2-((Cyclohexylacetyl)amino)ethyl)phenylsulfonyl)amino)carbonyl]-pyridin-2-carbonsäure-(2-methoxyethyl)amid

### Beispiel 373

5-[((4-(2-((2-Methylpropionyl)amino)ethyl)phenylsulfonyl)amino)carbonyl]-pyridin-2-carbonsäure-(2-methoxyethyl)amid

### Beispiel 374

5-[((3-(2-((2-Chlor-5-methoxybenzoyl)amino)ethyl)phenylsulfonyl)amino)carbonyl]-pyridin-2-carbonsäure-(2-methoxyethyl)amid

### Beispiel 375

5-[((3-(2-(Acetylamino)ethyl)phenylsulfonyl)amino)carbonyl]-pyridin-2-carbonsäure-(2-methoxyethyl)amid

### Beispiel 376

5-[((3-(2-(n-Butanoylamino)ethyl)phenylsulfonyl)amino)carbonyl]-pyridin-2-carbonsäure- (2-methoxyethyl)amid

### Beispiel 377

5-[((3-(2-(Benzoylamino)ethyl)phenylsulfonyl)amino)carbonyl]-pyridin-2-carbonsäure-(2-methoxyethyl)amid

### Beispiel 378

5-[((3-(2-((4-Chlorbenzoyl)amino)ethyl)phenylsulfonyl)amino)carbonyl]-pyridin-2-carbonsäure-(2-methoxyethyl)amid

### Beipsiel 379

5-[((3-(2-((5-Chlor-2-methoxybenzoyl)amino)ethyl)phenylsulfonyl)amino)carbonyl]-pyridin-2-carbonsäure-(2-methoxyethyl)amid

### Beispiel 380

5-[((3-(2-((3-Phenyl-n-propionyl)amino)ethyl)phenylsulfonyl)amino)carbonyl]-pyridin-2-carbonsäure-(2-methoxyethyl)amid

### Beispiel 381

5-[((3-(2-((2-Phenylacetyl)amino)ethyl)phenylsulfonyl)amino)carbonyl]-pyridin-2-carbonsäure- (2-methoxyethyl)amid

### Beispiel 382

5-[((3-(2-((Phenoxyacetyl)amino)ethyl)phenylsulfonyl)amino)carbonyl]-pyridin-2-carbonsäure-(2-methoxyethyl)amid

### Beispiel 383

5-[((3-(2-((4-Fluorbenzoyl)amino)ethyl)phenylsulfonyl)amino)carbonyl]-pyridin-2-carbonsäure-(2-methoxyethyl)amid

### Beispiel 384

5-[((3-(2-((4-Ethoxybenzoyl)amino)ethyl)phenylsulfonyl)amino)carbonyl]-pyridin-2-carbonsäure-(2-methoxyethyl)amid

### Beispiel 385

5-[((3-(2-((Cyclohexanoyl)amino)ethyl)phenylsulfonyl)amino)carbonyl]-pyridin-2-carbonsäure-(2-methoxyethyl)amid

### Beispiel 386

5-[((3-(2-((Cyclohexylacetyl)amino)ethyl)phenylsulfonyl)amino)carbonyl]-pyridin-2-carbonsäure-(2-methoxyethyl)amid

### Beispiel 387

5-[((3-(2-((2-Methylpropionyl)amino)ethyl)phenylsulfonyl)amino)carbonyl]-pyridin-2-carbonsäure-(2-methoxyethyl)amid

### Beispiel 388

5-[((4-(2-((2-Chlor-5-methoxybenzoyl)amino)ethyl)phenylsulfonyl)amino)carbonyl]-pyridin-2-carbonsäure-(2-methoxyethyl)amid

### Beispiel 389

5-[((4-(2-(Acetylamino)ethyl)phenylsulfonyl)amino)carbonyl]-pyridin-2-carbonsäure-(2-methoxyethyl)amid

### Beispiel 390

5-[((4-(2-(n-Butanoylamino)ethyl)phenylsulfonyl)amino)carbonyl]-pyridin-2-carbonsäure- (2-methoxyethyl)amid

### Beispiel 391

5-[((4-(2-(Benzoylamino)ethyl)phenylsulfonyl)amino)carbonyl]-pyridin-2-carbonsäure-(2-methoxyethyl)amid

### Beispiel 392

5-[((4-(2-((4-Chlorbenzoyl)amino)ethyl)phenylsulfonyl)amino)carbonyl]-pyridin-2-carbonsäure-(2-methoxyethyl)amid

### Beispiel 393

5-[((4-(2-((5-Chlor-2-methoxybenzoyl)amino)ethyl)phenylsulfonyl)amino)carbonyl]-pyridin-2-carbonsäure-(2-methoxyethyl)amid

### Beispiel 394

5-[((4-(2-((3-Phenyl-n-propionyl)amino)ethyl)phenylsulfonyl)amino)carbonyl]-pyridin-2-carbonsäure-(2-methoxyethyl)amid

### Beispiel 395

5-[((4-(2-((2-Phenylacetyl)amino)ethyl)phenylsulfonyl)amino)carbonyl]-pyridin-2-carbonsäure- (2-methoxyethyl)amid

### Beispiel 396

5-[((4-(2-((Phenoxyacetyl)amino)ethyl)phenylsulfonyl)amino)carbonyl]-pyridin-2-carbonsäure-(2-methoxyethyl)amid

### Beispiel 397

5-[((4-(2-((4-Fluorbenzoyl)amino)ethyl)phenylsulfonyl)amino)carbonyl]-pyridin-2-carbonsäure-(2-methoxyethyl)amid

### Beispiel 398

5-[((4-(2-((4-Ethoxybenzoyl)amino)ethyl)phenylsulfonyl)amino)carbonyl]-pyridin-2-carbonsäure-(2-methoxyethyl)amid

### Beispiel 399

5-[((4-(2-((Cyclohexanoyl)amino)ethyl)phenylsulfonyl)amino)carbonyl]-pyridin-2-carbonsäure-(2-methoxyethyl)amid

### Beispiel 400

5-[((4-(2-((Cyclohexylacetyl)amino)ethyl)phenylsulfonyl)amino)carbonyl]-pyridin-2-carbonsäure-(2-methoxyethyl)amid

### Beispiel 401

5-[((4-(2-((2-Methylpropionyl)amino)ethyl)phenylsulfonyl)amino)carbonyl]-pyridin-2-carbonsäure-(2-methoxyethyl)amid

### Beispiel 402

5-[((4-(2-((2-Chlor-5-methoxybenzoyl)amino)ethyl)phenylsulfonyl)amino)carbonyl]-pyridin-2-carbonsäure-(2-acetoxyethyl)amid

### Beispiel 403

5-[((4-(2-(Acetylamino)ethyl)phenylsulfonyl)amino)carbonyl]-pyridin-2-carbonsäure-(2-acetoxyethyl)amid

### Beispiel 404

5-[((4-(2-(n-Butanoylamino)ethyl)phenylsulfonyl)amino)carbonyl]-pyridin-2-carbonsäure-(2-acetoxyethyl)amid

### Beispiel 405

5-[((4-(2-(Benzoylamino)ethyl)phenylsulfonyl)amino)carbonyl]-pyridin-2-carbonsäure-(2-acetoxyethyl)amid

### Beispiel 406

5-[((4-(2-((4-Chlorbenzoyl)amino)ethyl)phenylsulfonyl)amino)carbonyl]-pyridin-2-carbonsäure- (2-acetoxyethyl)amid

### Beispiel 407

5-[((4-(2-((5-Chlor-2-methoxybenzoyl)amino)ethyl)phenylsulfonyl)amino)carbonyl]-pyridin-2-carbonsäure-(2-acetoxyethyl)amid

### Beispiel 408

5-[((4-(2-((3-Phenyl-n-propionyl)amino)ethyl)phenylsulfonyl)amino)carbonyl]-pyridin-2-carbonsäure-(2-acetoxyethyl)amid

### Beispiel 409

5-[((4-(2-((2-Phenylacetyl)amino)ethyl)phenylsulfonyl)amino)carbonyl]-pyridin-2-carbonsäure-(2-acetoxyethyl)amid

### Beispiel 410

5-[((4-(2-((Phenoxyacetyl)amino)ethyl)phenylsulfonyl)amino)carbonyl]-pyridin-2-carbonsäure-(2-acetoxyethyl)amid

### Beispiel 411

5-[((4-(2-((4-Fluorbenzoyl)amino)ethyl)phenylsulfonyl)amino)carbonyl]-pyridin-2-carbonsäure-(2-acetoxyethyl)amid

### Beispiel 412

5-[((4-(2-((4-Ethoxybenzoyl)amino)ethyl)phenylsulfonyl)amino)carbonyl]-pyridin-2-carbonsäure- (2-acetoxyethyl)amid

### Beispiel 413

5-[((4-(2-((Cyclohexanoyl)amino)ethyl)phenylsulfonyl)amino)carbonyl]-pyridin-2-carbonsäure-(2-acetoxyethyl)amid

### Beispiel 414

5-[((4-(2-((Cyclohexylacetyl)amino)ethyl)phenylsulfonyl)amino)carbonyl]-pyridin-2-carbonsäure-(2-acetoxyethyl)amid

### Beispiel 415

5-[((4-(2-((2-Methylpropionyl)amino)ethyl)phenylsulfonyl)amino)carbonyl]-pyridin-2-carbonsäure-(2-acetoxyethyl)amid

### Beispiel 416

5-[((4-(2-((2-Chlor-5-methoxybenzoyl)amino)ethyl)phenylsulfonyl)amino)carbonyl]-pyridin-2-carbonsäure- (2-(2-methylbenzoyl)oxyethyl)amid

### Beispiel 417

5-[((4-(2-(Acetylamino)ethyl)phenylsulfonyl)amino)carbonyl]-pyridin-2-carbonsäure-(2-(2-methylbenzoyl)oxyethyl)amid

### Beispiel 418

5-[((4-(2-(n-Butanoylamino)ethyl)phenylsulfonyl)amino)carbonyl]-pyridin-2-carbonsäure-(2-(2-methylbenzoyl)oxyethyl)amid

### Beispiel 419

5-[((4-(2-(Benzoylamino)ethyl)phenylsulfonyl)amino)carbonyl]-pyridin-2-carbonsäure-(2-(2-methylbenzoyl)oxyethyl)amid

### Beispiel 420

5-[((4-(2-((4-Chlorbenzoyl)amino)ethyl)phenylsulfonyl)amino)carbonyl]-pyridin-2-carbonsäure-(2-(2-methylbenzoyl)oxyethyl)amid

### Beispiel 421

5-[((4-(2-((5-Chlor-2-methoxybenzoyl)amino)ethyl)phenylsulfonyl)amino)carbonyl]-pyridin-2-carbonsäure-(2-(2-methylbenzoyl)oxyethyl)amid

### Beispiel 422

5-[((4-(2-((3-Phenyl-n-propionyl)amino)ethyl)phenylsulfonyl)amino)carbonyl]-pyridin-2-carbonsäure-(2-(2-methylbenzoyl)oxyethyl)amid

### Beispiel 423

5-[((4-(2-((2-Phenylacetyl)amino)ethyl)phenylsulfonyl)amino)carbonyl]-pyridin-2-carbonsäure-(2-(2-methylpropionyl)oxyethyl)amid

### Beispiel 424

5-[((4-(2-((Phenoxyacetyl)amino)ethyl)phenylsulfonyl)amino)carbonyl]-pyridin-2-carbonsäure-(2-(2-methylpropionyl)oxyethyl)amid

### Beispiel 425

5-[((4-(2-((4-Fluorbenzoyl)amino)ethyl)phenylsulfonyl)amino)carbonyl]-pyridin-2-carbonsäure-(2-(2-methylpropionyl)oxyethyl)amid

### Beispiel 426

5-[((4-(2-((4-Ethoxybenzoyl)amino)ethyl)phenylsulfonyl)amino)carbonyl]-pyridin-2-carbonsäure-(2-(2-methylpropionyl)oxyethyl)amid

### Beispiel 427

5-[((4-(2-((Cydohexanoyl)amino)ethyl)phenylsulfonyl)amino)carbonyl]-pyridin-2-carbonsäure-(2-(2-methylpropionyl)oxyethyl)amid

### Beispiel 428

5-[((4-(2-((Cyclohexylacetyl)amino)ethyl)phenylsulfonyl)amino)carbonyl]-pyridin-2-carbonsäure-(2-(2-methylpropionyl)oxyethyl)amid

### Beispiel 429

5-[((4-(2-((2-Methylpropionyl)amino)ethyl)phenylsulfonyl)amino)carbonyl]-pyridin-2-carbonsäure-(2-(2-methylpropionyl)oxyethyl)amid

### Beispiel 430

5-[((4-(2-((2-Ethylbutanoyl)amino)ethyl)phenylsulfonyl)amino)carbonyl]-pyridin-2-carbonsäure- (2-hydroxyethyl)amid

### Beispiel 431

5-[((4-(2-((2-Ethylbutanoyl)amino)ethyl)phenylsulfonyl)amino)carbonyl]-pyridin-2-carbonsäure- (2-methoxyethyl)amid

### Beispiel 432

5-[((4-(2-((4-n-Butoxybenzoyl)amino)ethyl)phenylsulfonyl)amino)carbonyl]-pyridin-2-carbonsäure- (2-hydroxyethyl)amid

### Beispiel 433

5-[((4-(2-((2-Chlor-5-methoxybenzoyl)amino)ethyl)phenylsulfonyl)amino)carbonyl]-pyridin-2-carbonsäure-(glycylmethylester)amid
a) 5-[((4-(2-((2-Chlor-5-methoxybenzoyl)amino)ethyl)phenylsulfonyl)amino)carbonyl]-pyridin-2-carbonsäure
   0,8g (1,36 mmol) des Methylesters aus Beispiel 332 a) wurden analog Beispiel 202 a) mit 30 ml 1n methanolischer NaOH verseift. Nach dem Einengen i. Vak. wurde der Rückstand in Tetrahydrofuran gelöst, mit Zn wäßriger HCI angesäuert, eingeengt, der Rückstand mit Wasser behandelt und abgesaugt. Man isolierte 0,75g Produkt, Fp. 149°C (Zers.)
b) 0,7g (1,35 mmol) des vorstehenden Pyridin-2-carbonsäure-Derivats wurden analog Beispiel 202 b) in 100 ml wasserfreiem Acetonitril mit 0,19 (1,5 mmol) Glycinmethylester-Hydrochlorid, 0,4 ml (3 mmol) N-Ethylmorpholin, 0,33 g (1,5 mmol) 1-Hydroxybenzotriazol und 0,31 g (1,5 mmol) N.N'-Dicyclohexylcarbodiimid umgesetzt.
   Dann wurde vom Umgelösten abfiltriert, das Filtrat eingeengt, der Rückstand in 1,4-Dioxan aufgenommen, mit 2n wäßriger HCI angesäuert und i. Vak. eingeengt. Das kristalline Produkt wurde abgesaugt, gewaschen und getrocknet. Diese Rohprodukt wurde anschließend mit Dichlormethan/Methanol (19:1) an Kieselgel chromatographiert. Entsprechende Fraktionen wurden eingedampft und der Rückstand aus heißem Methanol kristallisiert, abgesaugt, mit Methanol gewaschen und getrocknet. Man erhielt 0,51g der Titelverbindung als farblos kristallines Produkt, Fp. 192-194°C.

### Beispiel 434

5-[((4-(2-(Acetylamino)ethyl)phenylsulfonyl)amino)carbonyl]-pyridin-2-carbonsäure-(glycylmethylester)amid

### Beispiel 435

5-[((4-(2-(n-Hexanoylamino)ethyl)phenylsulfonyl)amino)carbonyl]-pyridin-(glycylmethylester)amid
Fp. 128-130° C (Ethylacetat)

### Beispiel 436

5-[((4-(2-(Benzoylamino)ethyl)phenylsulfonyl)amino)carbonyl]-pyridin-2-carbonsäure-(glycylmethylester)amid

### Beispiel 437

5-[((4-(2-((4-Chlorbenzoyl)amino)ethyl)phenylsulfonyl)amino)carbonyl]-pyridin-2-carbonsäure-(glycylmethylester)amid

### Beispiel 438

5-[((4-(2-((3,4-Dimethoxyphenyl)propionyl)amino)ethyl)phenylsulfonyl)amino)carbonyl]-pyridin-2-carbonsäure-(glycylmethylester)amid
Fp. 139-141° C (nach Chromatographie mit Ethylacetat/Methanol (9:1) an Kieselgel)

### Beispiel 439

5-[((4-(2-((3-Phenyl-n-propionyl)amino)ethyl)phenylsulfonyl)amino)carbonyl]-pyridin-2-carbonsäure-(glycylmethylester)amid

### Beispiel 440

5-[((4-(2-((2-Phenylacetyl)amino)ethyl)phenylsulfonyl)amino)carbonyl]-pyridin-2-carbonsäure-(glycylmethylester)amid

### Beispiel 441

5-[((4-(2-((Phenoxyacetyl)amino)ethyl)phenylsulfonyl)amino)carbonyl]-pyridin-2-carbonsäure-(glycylmethylester)amid

### Beispiel 442

5-[((4-(2-((4-Fluorbenzoyl)amino)ethyl)phenylsulfonyl)amino)carbonyl]-pyridin-2-carbonsäure-(glycylmethylester)amid

### Beispiel 443

5-[((4-(2-((3,4-Diethoxybenzoyl)amino)ethyl)phenylsulfonyl)amino)carbonyl]-pyridin-2-carbonsäure-(glycylethylester)amid
Fp. 215-217° C (Wasser)

### Beispiel 444

5-[((4-(2-((Cyclohexanoyl)amino)ethyl)phenylsulfonyl)amino)carbonyl]-pyridin-2-carbonsäure-(glycylmethylester)amid

### Beispiel 445

5-[((4-(2-((Cyclohexylacetyl)amino)ethyl)phenylsulfonyl)amino)carbonyl]-pyridin-2-carbonsäure-(glycylmethylester)amid
Fp. 178-179° C (Methanol/Diisopropylether)

### Beispiel 446

5-[((4-(2-((2-Methylpropionyl)amino)ethyl)phenylsulfonyl)amino)carbonyl]-pyridin-2-carbonsäure-(glycylmethylester)amid
Fp. 197-199° C (nach Chromatographie mit Ethylacetat/Methanol (9:1) an Kieselgel)

### Beispiel 447

5-[((4-(2-(4-Methylpentanoyl)amino)ethyl)phenylsulfonyl)amino)carbonyl]-pyridin-2-carbonsäure-(glycylmethylester)amid
Fp. 160-162 (nach Chromatographie mit Ethylacetat/Methanol an Kieselgel)

### Beispiel 448

5-[((4-(2-((2-Chlor-5-methoxybenzoyl)amino)ethyl)phenylsulfonyl)amino)carbonyl]-pyridin-2-carbonsäure-(glycyl-2-propylester)amid

### Beispiel 449

5-[((4-(2-(Acetylamino)ethyl)phenylsulfonyl)amino)carbonyl]-pyridin-2-carbonsäure-(glycyl-2-propylester)amid

### Beispiel 450

5-[((4-(2-(n-Butanoylamino)ethyl)phenylsulfonyl)amino)carbonyl]-pyridin-(glycyl-2-propylester)amid

### Beispiel 451

5-[((4-(2-(Benzoylamino)ethyl)phenylsulfonyl)amino)carbonyl]-pyridin-2-carbonsäure-(glycyl-2-propylester)amid

### Beispiel 452

5-[((4-(2-((4-Chlorbenzoyl)amino)ethyl)phenylsulfonyl)amino)carbonyl]-pyridin-2-carbonsäure-(glycyl-2-propylester)amid

### Beispiel 453

5-[((4-(2-((5-Chlor-2-methoxybenzoyl)amino)ethyl)phenylsulfonyl)amino)carbonyl]-pyridin-2-carbonsäure-(glycyl-2-propylester)amid

### Beispiel 454

5-[((4-(2-((3-Phenyl-n-propionyl)amino)ethyl)phenylsulfonyl)amino)carbonyl]-pyridin-2-carbonsäure-(glycyl-2-propylester)amid

### Beispiel 455

5-[((4-(2-((2-Phenylacetyl)amino)ethyl)phenylsulfonyl)amino)carbonyl]-pyridin-2-carbonsäure-(glycyl-2-propylester)amid

### Beispiel 456

5-[((4-(2-((Phenoxyacetyl)amino)ethyl)phenylsulfonyl)amino)carbonyl]-pyridin-2-carbonsäure-(glycyl-2-propylester)amid

### Beispiel 457

5-[((4-(2-((4-Fluorbenzoyl)amino)ethyl)phenylsulfonyl)amino)carbonyl]-pyridin-2-carbonsäure-(glycyl-2-propylester)amid

### Beispiel 458

5-[((4-(2-((4-Ethoxybenzoyl)amino)ethyl)phenylsulfonyl)amino)carbonyl]-pyridin-2-carbonsäure-(glycyl-2-propylester)amid

### Beispiel 459

5-[((4-(2-((Cyclohexanoyl)amino)ethyl)phenylsulfonyl)amino)carbonyl]-pyridin-2-carbonsäure-(glycyl-2-propylester)amid

### Beispiel 460

5-[((4-(2-((Cyclohexylacetyl)amino)ethyl)phenylsulfonyl)amino)carbonyl]-pyridin-2-carbonsäure-(glycyl-2-propylester)amid

### Beispiel 461

5-[((4-(2-(2-Methylpropionyl)amino)ethyl)phenylsulfonyl)amino)carbonyl]-pyridin-2-carbonsäure-(glycyl-2-propylester)amid

### Beispiel 462

5-[((4-(2-(2-Ethylbutanonyl)amino)ethyl)phenylsulfonyl)amino)carbonyl]-pyridin-2-carbonsäure-(glycyl-2-propylester)amid

### Beispiel 463

5-[((4-(2-((2-Chlor-5-methoxybenzoyl)amino)ethyl)phenylsulfonyl)amino)carbonyl]-pyridin-2-carbonsäure-(glycylbenzylester)amid

### Beispiel 464

5-[((4-(2-(Acetylamino)ethyl)phenylsulfonyl)amino)carbonyl]-pyridin-2-carbonsäure-(glycylbenzylester)amid

### Beispiel 465

5-[((4-(2-(n-Butanoylamino)ethyl)phenylsulfonyl)amino)carbonyl]-pyridin-(glycylbenzylester)amid

### Beispiel 466

5-[((4-(2-(Benzoylamino)ethyl)phenylsulfonyl)amino)carbonyl]-pyridin-2-carbonsäure-(glycylbenzylester)amid

### Beispiel 467

5-[((4-(2-((4-Chlorbenzoyl)amino)ethyl)phenylsulfonyl)amino)carbonyl]-pyridin-2-carbonsäure-(glycylbenzylester)amid

### Beispiel 468

5-[((4-(2-((5-Chlor-2-methoxybenzoyl)amino)ethyl)phenylsulfonyl)amino)carbonyl]-pyridin-2-carbonsäure-(glycylbenzylester)amid

### Beispiel 469

5-[((4-(2-((3-Phenyl-n-propionyl)amino)ethyl)phenylsulfonyl)amino)carbonyl]-pyridin-2-carbonsäure-(glycylbenzylester)amid

### Beispiel 470

5-[((4-(2-((2-Phenylacetyl)amino)ethyl)phenylsulfonyl)amino)carbonyl]-pyridin-2-carbonsäure-(glycylbenzylester)amid

### Beispiel 471

5-[((4-(2-((Phenoxyacetyl)amino)ethyl)phenylsulfonyl)amino)carbonyl]-pyridin-2-carbonsäure-(glycylbenzylester)amid

### Beispiel 472

5-[((4-(2-((4-Fluorbenzoyl)amino)ethyl)phenylsulfonyl)amino)carbonyl]-pyridin-2-carbonsäure-(glycylbenzylester)amid

### Beispiel 473

5-[((4-(2-((4-Ethoxybenzoyl)amino)ethyl)phenylsulfonyl)amino)carbonyl]-pyridin-2-carbonsäure-(glycylbenzylester)amid

### Beispiel 474

5-[((4-(2-((Cydohexanoyl)amino)ethyl)phenylsulfonyl)amino)carbonyl]-pyridin-2-carbonsäure-(glycylbenzylester)amid

### Beispiel 475

5-[((4-(2-((Cyclohexylacetyl)amino)ethyl)phenylsulfonyl)amino)carbonyl]-pyridin-2-carbonsäure-(glycylbenzylester)amid

### Beispiel 476

5-[((4-(2-(2-Methylpropionyl)amino)ethyl)phenylsulfonyl)amino)carbonyl]-pyridin-2-carbonsäure-(glycylbenzylester)amid

### Beispiel 477

5-[((4-(2-(2-Ethylbutanonyl)amino)ethyl)phenylsulfonyl)amino)carbonyl]-pyridin-2-carbonsäure-(glycylbenzylester)amid

### Beispiel 478

5-[((4-(2-((2-Chlor-5-methoxybenzoyl)amino)ethyl)phenylsulfonyl)amino)carbonyl]-pyridin-2-carbonsäure-glycylamid

### Beispiel 479

5-[((4-(2-(Acetylamino)ethyl)phenylsulfonyl)amino)carbonyl]-pyridin-2-carbonsäure-glycylamid

### Beispiel 480

5-[((4-(2-(n-Hexanoylamino)ethyl)phenylsulfonyl)amino)carbonyl]-pyridin-glycylamid Fp. 115-116° C (aus Tetrahydrofuran/wäßrige Salzsäure)

### Beispiel 481

5-[((4-(2-(Benzoylamino)ethyl)phenylsulfonyl)amino)carbonyl]-pyridin-2-carbonsäure-glycylamid

### Beispiel 482

5-[((4-(2-((4-Chlorbenzoyl)amino)ethyl)phenylsulfonyl)amino)carbonyl]-pyridin-2-carbonsäure-glycylamid

### Beispiel 483

5-[((4-(2-((3,4-Dimethoxybenzoyl)amino)ethyl)phenylsulfonyl)amino)carbonyl]-pyridin-2-carbonsäure-glycylamid

### Beispiel 484

5-[((4-(2-((3-Phenyl-n-propionyl)amino)ethyl)phenylsulfonyl)amino)carbonyl]-pyridin-2-carbonsäure-glycylamid

### Beispiel 485

5-[((4-(2-((2-Phenylacetyl)amino)ethyl)phenylsulfonyl)amino)carbonyl]-pyridin-2-carbonsäure-glycylamid

### Beispiel 486

5-[((4-(2-((Phenoxyacetyl)amino)ethyl)phenylsulfonyl)amino)carbonyl]-pyridin-2-carbonsäure-glycylamid

### Beispiel 487

5-[((4-(2-((4-Fluorbenzoyl)amino)ethyl)phenylsulfonyl)amino)carbonyl]-pyridin-2-carbonsäure-glycylamid

### Beispiel 488

5-[((4-(2-((3,4-Diethoxybenzoyl)amino)ethyl)phenylsulfonyl)amino)carbonyl]-pyridin-2-carbonsäure-glycylamid
Fp. > 230° C (aus wäßriger Salzsäure)

### Beispiel 489

5-[((4-(2-((Cyclohexanoyl)amino)ethyl)phenylsulfonyl)amino)carbonyl]-pyridin-2-carbonsäure-glycylamid

### Beispiel 490

5-[((4-(2-((Cyclohexylacetyl)amino)ethyl)phenylsulfonyl)amino)carbonyl]-pyridin-2-carbonsäure-glycylamid
Fp. 154-156° C (aus wäßriger Salzsäure)

### Beispiel 491

5-[((4-(2-(2-Methylpropionyl)amino)ethyl)phenylsulfonyl)amino)carbonyl]-pyridin-2-carbonsäure-glycylamid
Fp. 269-271 ° C (aus wäßriger Salzsäure)

### Beispiel 492

5-[((4-(2-(4-Methylpentanoyl)amino)ethyl)phenylsulfonyl)amino)carbonyl]-pyridin-2-carbonsäure-glycylamid
Fp. 126-128° C (aus wäßriger Salzsäure)

### Beispiel 493

5-[((3-(2-((2-Chlor-5-methoxybenzoyl)amino)ethyl)phenylsulfonyl)amino)carbonyl]-pyridin-2-carbonsäure-(glycylmethylester)amid

### Beispiel 494

5-[((3-(2-(Acetylamino)ethyl)phenylsulfonyl)amino)carbonyl]-pyridin-2-carbonsäure-(glycylmethylester)amid

### Beispiel 495

5-[((3-(2-(n-Bulanoylamino)ethyl)phenylsulfonyl)amino)carbonyl]-pyridin-(glycylmethylester)amid

### Beispiel 496

5-[((3-(2-(Benzoylamino)ethyl)phenylsulfonyl)amino)carbonyl]-pyridin-2-carbonsäure-(glycylmethylester)amid

### Beispiel 497

5-[((3-(2-((4-Chlorbenzoyl)amino)ethyl)phenylsulfonyl)amino)carbonyl]-pyridin-2-carbonsäure-(glycylmethylester)amid

### Beispiel 498

5-[((3-(2-((5-Chlor-2-methoxybenzoyl)amino)ethyl)phenylsulfonyl)amino)carbonyl]-pyridin-2-carbonsäure-(glycylmethylester)amid

### Beispiel 499

5-[((3-(2-((3-Phenyl-n-propionyl)amino)ethyl)phenylsulfonyl)amino)carbonyl]-pyridin-2-carbonsäure-(glycylmethylester)amid

### Beispiel 500

5-[((3-(2-((3-Phenylacetyl)amino)ethyl)phenylsulfonyl)amino)carbonyl]-pyridin-2-carbonsäure-(glycylmethylester)amid

### Beispiel 501

5-[((4-(3-((Phenoxyacetyl)amino)ethyl)phenylsulfonyl)amino)carbonyl]-pyridin-2-carbonsäure-(glycylmethylester)amid

### Beispiel 502

5-[((3-(2-((4-Fluorbenzoyl)amino)ethyl)phenylsulfonyl)amino)carbonyl]-pyridin-2-carbonsäure-(glycylmethylester)amid

### Beispiel 503

5-[((3-(2-((4-Ethoxybenzoyl)amino)ethyl)phenylsulfonyl)amino)carbonyl]-pyridin-2-carbonsäure-(glycylmethylester)amid

### Beispiel 504

5-[((3-(2-((Cyclohexanoyl)amino)ethyl)phenylsulfonyl)amino)carbonyl]-pyridin-2-carbonsäure-(glycylmethylester)amid

### Beispiel 505

5-[((3-(2-((Cyclohexylacetyl)amino)ethyl)phenylsulfonyl)amino)carbonyl]-pyridin-2-carbonsäure-(glycylmethylester)amid

### Beispiel 506

5-[((3-(2-(2-Methylpropionyl)amino)ethyl)phenylsulfonyl)amino)carbonyl]-pyridin-2-carbonsäure-(glycylmethylester)amid

### Beispiel 507

5-[((3-(2-(2-Ethylbutanonyl)amino)ethyl)phenylsulfonyl)amino)carbonyl]-pyridin-2-carbonsäure-(glycylmethylester)amid

### Beispiel 508

5-[((4-((4-Phenyl-n-butanoyl)amino)phenylsulfonyl)amino)carbonyl]-pyridin-2-carbonsäure-(2-hydroxyethyl)amid
a)4-((Phenyl-n-butanoyl)amino)benzolsulfonsäureamid
   16,5g (0,1 mol) 4-Phenylbuttersäure wurden in 300ml wasserfreiem Tetrahydrofuran bei 0°C mit 11,1g (0,11 mol, 15,2ml) Triethylamin versetzt. Nach 30 min. tropfte man bei 0°C 12g (0,11 mol, 10,5ml) Chlorameisensäureethylester zu. Zu dieser dicken Suspension wurde bei -10°C eine Lösung von 18,1g (0,105 mol) 4-Aminobenzolsulfonamid in 150 ml wasserfreiem Tetrahydrofuran hinzugetropft. Es wurde 1 h bei 0°C, 1h bei 25°C gerührt, i.Vak. eingeengt, der Rückstand mit wäßriger Salzsäure behandelt. Das kristalline Rohprodukt wird mit Wasser gewaschen und aus 250ml Methanol umkristallisiert; Ausbeute 18g; Fp. 166-168°C.
b) 5-[((4-((4-Phenyl-n-butanoyl)amino)phenylsulfonyl)amino)carbonyl]-pyridin-2-carbonsäure-methylester
   Analog Beispiel 112b) werden 1,8g (10mmol) Pyridin-2,5-dicarbonsäure-2-methylester in 300ml Acetonitril mit 3,2g (10mmol) des vorstehenden Benzolsulfonamids, 2,1g (10mmol) N,N'-Dicyclohexylcarbodiimid und 1,2g (10 mmol) 4-N,N-Dimethylaminopyridin umgesetzt.
   Man filtrierte vom Ungelösten ab, engte ein, versetzte mit wäßriger Salzsäure (pH1) und saugt das feinkristalline Produkt ab. Dieses wird in N,N-Dimethylformamid gelöst und bis zur beginnenden Trübung mit Wasser versetzt. Das kristalline Rohprodukt wird mit Wasser gewaschen und getrocknet; 3,3g; Fp. 258-264°C.
   Nach Chromatographie mit Ethylacetat Methanol (3:1) an Kieselgel werden entsprechende Fraktionen eingedampft und aus Methanol umkristallisiert. Man isolierte 1,4g farblos kristallines Produkt; Fp. 258°C (u.Zers.)
c) Die Titelverbindung wurde erhalten, indem 0,3g (0,62 mmol) des vorstehenden Methylesters in 5 ml 2-Aminoethanol 2h bei 80°C gerührt wurden. Das überschüssige Reagent wurde i.Vak. abdestilliert, der Rückstand in wenig Tetrahydrofuran gelöst, mit 2 n wäßriger HCI angesäuert, i.Vak. eingeengt, die kristallinie Fällung abgesaugt, mit Wasser gewaschen und getrocknet. Man erhielt 0,21g der Titelverbindung; Fp. 278-280°C.

### Beispiel 509

5-[((4-(3-Phenyl-n-propionylamino)phenylsulfonyl)amino)carbonyl]-pyridin-2-carbonsäure-(2-hydroxyethyl)amid

### Beispiel 510

5-[((4-(2-Phenylacetylamino)phenylsulfonyl)amino)carbonyl]-pyridin-2-carbonsäure-(2-hydroxyethyl)amid

### Beispiel 511

5[((4-(Benzoylamino)phenylsulfonyl)amino)carbonyl]-pyridin-2-carbonsäure-(2-hydroxyethyl)amid

### Beispiel 512

5[((4-(Acetylamino)phenylsulfonyl)amino)carbonyl]-pyridin-2-carbonsäure-(2-hydroxyethyl)amid

### Beispiel 513

5-[((4-(n-Propionylamino)phenylsulfonyl)amino)carbonyl]-pyridin-2-carbonsäure-(2-hydroxyethyl)amid

### Beispiel 514

5-[((4-(n-Hexanoylamino)phenylsulfonyl)amino)carbonyl]-pyridin-2-carbonsäure-(2-hydroxyethyl)amid

### Beispiel 515

5-[((4-((2-Phenoxyacetyl)amino)phenylsulfonyl)amino)carbonyl]-pyridin-2-carbonsäure-(2-hydroxyethyl)amid

### Beispiel 516

5-[((4-(n-Butanoylamino)phenylsulfonyl)amino)carbonyl]-pyridin-2-carbonsäure-(2-hydroxyethyl)amid

### Beispiel 517

5-[((4-(Cyclohexanoylamino)phenylsulfonyl)amino)carbonyl]-pyridin-2-carbonsäure-(2-hydroxyethyl)amid

### Beispiel 518

5-[((4-(Cyclohexylacetyl)amino)phenylsulfonyl)amino)carbonyl]-pyridin-2-carbonsäure-(2-hydroxyethyl)amid

### Beispiel 519

5-[((4-(4-Phenyl-n-butanoylamino)phenylsulfonyl)amino)carbonyl]-pyridin-2-carbonsäure-(2-methoxyethyl)amid

### Beispiel 520

5-[((4-(3-Phenyl-n-propionylamino)phenylsulfonyl)amino)carbonyl]-pyridin-2-carbonsäure- (2-methoxyethyl)amid

### Beispiel 521

5-[((4-(2-Phenylacetylamino)phenylsulfonyl)amino)carbonyl]-pyridin-2-carbonsäure-(2-methoxyethyl)amid

### Beispiel 522

5[((4-(Benzoylamino)phenylsulfonyl)amino)carbonyl]-pyridin-2-carbonsäure-(2-methoxyethyl)amid

### Beispiel 523

5[((4-(Acetylamino)phenylsulfonyl)amino)carbonyl]-pyridin-2-carbonsäure-(2-methoxyethyl)amid

### Beispiel 524

5-[((4-(n-Propionylamino)phenylsulfonyl)amino)carbonyl]-pyridin-2-carbonsäure-(2-methoxyethyl)amid

### Beispiel 525

5-[((4-(n-Hexanoylamino)phenylsulfonyl)amino)carbonyl]-pyridin-2-carbonsäure-(2-methoxyethyl)amid

### Beispiel 526

5-[((4-((2-Phenoxyacetyl)amino)phenylsulfonyl)amino)carbonyl]-pyridin-2-carbonsäure- (2-methoxyethyl)amid

### Beispiel 527

5-[((4-(n-Butanoylamino)phenylsulfonyl)amino)carbonyl]-pyridin-2-carbonsäure-(2-methoxyethyl)amid

### Beispiel 528

5-[((4-(Cyclohexanoylamino)phenylsulfonyl)amino)carbonyl]-pyridin-2-carbonsäure-(2-methoxyethyl)amid

### Beispiel 529

5-[((4-(Cyclohexylacetyl)amino)phenylsulfonyl)amino)carbonyl]-pyridin-2-carbonsäure-(2-methoxyethyl)amid

### Beispiel 530

5-[(((4-Phenyl-n-butyl)sulfonyl)amino)carbonyl]-pyridin-2-carbonsäure-(glycyl-2-propylester)amid

### Beispiel 531

5-[(((2-Phenoxyethyl)sulfonyl)amino)carbonyl]-pyridin-2-carbonsäure-(glycyl-2-propylester)amid

### Beispiel 532

5-[((2-(4-Fluorphenoxy)ethylsulfonyl)amino)carbonyl]-pyridin-2-carbonsäure-(glycyl-2-propylester)amid

### Beispiel 533

5-[((Phenylmethylsulfonyl)amino)carbonyl]-pyridin-2-carbonsäure-(glycylbenzylester)amid

### Beispiel 534

5-[(((2-Phenylethyl)sulfonyl)amino)carbonyl]-pyridin-2-carbonsäure-(glycylbenzylester)amid

### Beispiel 535

5-[(((2-(4-Fluorphenyl)ethyl)sulfonyl)amino)carbonyl]-pyridin-2-carbonsäure-(glycylbenzylester)amid

### Beispiel 536

5-[(((2-(4-Methoxyphenyl)ethyl)sulfonyl)amino)carbonyl]-pyridin-2-carbonsäure-(glycylbenzylester)amid

### Beispiel 537

5-[(((3-Phenyl-n-propyl)sulfonyl)amino)carbonyl]-pyridin-2-carbonsäure-(glycylbenzylester)amid

### Beispiel 538

5-[(((4-Phenyl-n-butyl)sulfonyl)amino)carbonyl]-pyridin-2-carbonsäure-(glycylbenzylester)amid

### Beispiel 539

5-[(((2-Phenoxyethyl)sulfonyl)amino)carbonyl]-pyridin-2-carbonsäure-(glycylbenzylester)amid

### Beispiel 540

5-[(((2-(4-Fluorphenoxy)ethyl)sulfonyl)amino)carbonyl]-pyridin-2-carbonsäure-(glycylbenzylester)amid

### Beispiel 541

5-[((Phenylmethylsulfonyl)amino)carbonyl]-pyridin-2-carbonsäure-glycylamid

### Beispiel 542

5-[(((2-Phenylethyl)sulfonyl)amino)carbonyl]-pyridin-2-carbonsäure-glycylamid

### Beispiel 543

5-[(((2-(4-Fluorphenyl)ethylsulfonyl)amino)carbonyl]-pyridin-2-carbonsäure-glycylamid

### Beispiel 544

5-[(((2-(4-Methoxyphenyl)ethyl)sulfonyl)amino)carbonyl]-pyridin-2-carbonsäure-glycylamid

### Beispiel 545

5-[(((3-Phenyl-n-propyl)sulfonyl)amino)carbonyl]-pyridin-2-carbonsäure

### Beispiel 546

5-[(((4-Phenyl-n-butyl)sulfonyl)amino)carbonyl]-pyridin-2-carbonsäure-glycylamid

### Beispiel 547

5-[(((2-Phenoxyethyl)sulfonyl)amino)carbonyl]-pyridin-2-carbonsäure-glycylamid

### Beispiel 548

5-[(((2-(4-Fluorphenoxy)ethyl)sulfonyl)amino)carbonyl]-pyridin-2-carbonsäure-glycylamid

### Beispiel 549

5-[((Phenylmethylsulfonyl)amino)carbonyl]-pyridin-2-carbonsäure-(glycyl-3-pentylester)amid

### Beispiel 550

5-[(((2-Phenylethyl)sulfonyl)amino)carbonyl]-pyridin-2-carbonsäure-(glycyl-3-pentylester)amid

### Beispiel 551

5-[(((2-(4-Fluorphenyl)ethyl)sulfonyl)amino)carbonyl]-pyridin-2-carbonsäure-(glycyl-3-pentylester)amid

### Beispiel 552

5-[((2-(4-Methoxyphenyl)ethylsulfonyl)amino)carbonyl]-pyridin-2-carbonsäure-(glycyl-3-pentylester)amid

### Beispiel 553

5-[(((3-Phenyl-n-propyl)sulfonyl)amino)carbonyl]-pyridin-2-carbonsäure-(glycyl-4-heptylester)amid

### Beispiel 554

5-[(((4-Phenyl-n-butyl)sulfonyl)amino)carbonyl]-pyridin-2-carbonsäure-(glycyl-R-2-butylester)amid

### Beispiel 555

5-[(((2-Phenoxyethyl)sulfonyl)amino)carbonyl]-pyridin-2-carbonsäure-(glycyl-n-octylester)amid

### Beispiel 556

5-[(((2-(4-Fluorphenoxy)ethyl)sulfonyl)amino)carbonyl]-pyridin-2-carbonsäure-(glycyl-cyclohexylester)amid

### Beispiel 557

5-[((4-(2-((3,4-Dimethoxybenzoyl)amino)ethyl)phenylsulfonyl)amino)carbonyl]-pyridin-2-carbonsäure-(2-hydroxyethyl)amid

### Beispiel 558

5-[((4-(2-((3,4-Dimethoxybenzoyl)amino)ethyl)phenylsulfonyl)amino)carbonyl]-pyridin-2-carbonsäure-(2-acetoxyethyl)amid

### Beispiel 559

5-[((4-(2-((2,5-Dimethoxybenzoyl)amino)ethyl)phenylsulfonyl)amino)carbonyl]-pyridin-2-carbonsäure-(2-hydroxyethyl)amid

### Beispiel 560

5-[((4-(2-((3,4-Dimethoxybenzoyl)amino)ethyl)phenylsulfonyl)amino)carbonyl]-pyridin-2-carbonsäure-(2-methoxyethyl)amid

### Beispiel 561

5-[((4-(2-((3,4-Dimethoxybenzoyl)amino)ethyl)phenylsulfonyl)amino)carbonyl]-pyridin-2-carbonsäure-(2-ethoxyethyl)amid

### Beispiel 562

5-[((4-(2-((2,5-Dimethoxybenzoyl)amino)ethyl)phenylsulfonyl)amino)carbonyl]-pyridin-2-carbonsäure-(2-methoxyethyl)amid

### Beispiel 563

5-[((4-(2-((3,4-Dimethoxybenzoyl)amino)ethyl)phenylsulfonyl)amino)carbonyl]-pyridin-2-carbonsäure-(glycylmethylester)amid

### Beispiel 564

5-[((4-(2-((3,4-Dimethoxybenzoyl)amino)ethyl)phenylsulfonyl)amino)carbonyl]-pyridin-2-carbonsäure-(glycylethylester)amid

### Beispiel 565

5-[((4-(2-((2,5-Dimethoxybenzoyl)amino)ethyl)phenylsulfonyl)amino)carbonyl]-pyridin-2-carbonsäure-(glycylmethyl-ester)amid

### Beispiel 566

5-[((4-(2-((3,4-Dimethoxybenzoyl)amino)ethyl)phenylsulfonyl)amino)carbonyl]-pyridin-2-carbonsäure-(glycyl-2-propylester)amid

### Beispiel 567

5-[((4-(2-((3,4-Dimethoxybenzoyl)amino)ethyl)phenylsulfonyl)amino)carbonyl]-pyridin-2-carbonsäure-(glycyl-3-pentylester)amid

### Beispiel 568

5-[((4-(2-((2,5-Dimethoxybenzoyl)amino)ethyl)phenylsulfonyl)amino)carbonyl]-pyridin-2-carbonsäure-(glycyl-2-propylester)amid

### Beispiel 569

5-[((4-(2-((3,4-Dimethoxybenzoyl)amino)ethyl)phenylsulfonyl)amino)carbonyl]-pyridin-2-carbonsäure-glycylamid

### Beispiel 570

5-[((4-(2-((3,4-Dimethoxybenzoyl)amino)ethyl)phenylsulfonyl)amino)carbonyl]-pyridin-2-carbonsäure-(glycyl-cyclohexylester)amid

### Beispiel 571

5-[((4-(2-((2,5-Dimethoxybenzoyl)amino)ethyl)phenylsulfonyl)amino)carbonyl]-pyridin-2-carbonsäure-glycylamid

## Patentansprüche

1. Sulfonamidocarbonylpyridin-2-carbonsäureamide der allgemeinen Formel I, 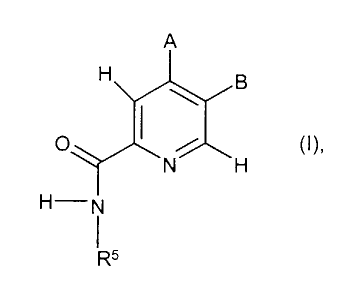 worin
A = H und B = -CO-NR⁶-R⁷ oder
B = H und A = -CO-NR⁶R⁷ bedeutet und
R⁶ Wasserstoff oder ein 1 oder 2-wertiges physiologisch verwendbares Kation,
R⁷ einen Rest der Formel II bedeutet
-SO₂-C-U-D-W (II),
in welchem
C eine Bindung oder (C₁-C₄)-Alkandiyl,
U eine Bindung oder -O- bedeutet,
D eine Bindung oder (C₁-C₄)-Alkandiyl,
W einen Phenylrest bedeutet, wobei U nur dann in der Bedeutung von -O- steht, wenn C nicht eine Bindung bedeutet oder wenn D nicht eine Bindung bedeutet und
W substituiert ist durch Wasserstoff, Fluor, Chlor, (C₁-C₆)-Alkyl, (C₃-C₈)-Cycloalkyl, (C₆-C₁₂)-Aryl, (C₇-C₁₆)-Aralkyl, C₃-C₁₂)-Alkenyl, (C₁-C₆)-Alkoxy, Phenoxy, -O-[-CH₂]_{X}C_{f}H_{(2f+1-g)}F_{g},
Carbamoyl, N-(C₁-C₁₀)-Alkylcarbamoyl, N,N-Di(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-phenylcarbamoyl, N-(C₁-C₈)-Alkyl-N-(C₇-C₁₁)-Phenylalkylcarbamoyl, N-(C₁-C₈)-Alkyl-N-Phenylalkylcarbamoyl, N-((C₁-C₁₀)-Alkoxy-(C₁-C₈)alkyl)carbamoyl,
N-Phenoxycarbamoyl,
N-((C₇-C₁₆)-Phenylalkyloxy-(C₁-C₈)alkyl)carbamoyl,
N-(C₁-C₈)-Alkyl-N-(C₁-C₆)-Alkoxy-(C₁-C₈)alkyl)carbamoyl,
N-(C₁-C₈)-Alkyl-N-(Phenoxy-(C₁-C₈)alkyl)carbamoyl,
N-(C₁-C₈)-Alkyl-N-((C₇-C₁₆)-Phenylalkyloxy-(C₁-C₈)alkyl)carbamoyl,
(C₁-C₈)-Akanoylamino, (C₃-C₈)-Cycloalkanoylamino, Phenylamino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₈)-alkyl, (C₃-C₈)-Cycloalkanoylamino-(C₁-C₈)alkyl, Phenylamino-(C₁-C₈)-alkyl, (C₇-C₁₁)-Phenylalkanoylamino-(C₁-C₈)-alkyl,
wobei die Reste, die einen Arylrest enthalten, ihrerseits am Aryl substituiert sein können mit 1, 2 oder 3 gleichen oder verschiedenen Substituenten aus der Reihe Hydroxy, Carboxyl, (C₁-C₄)-Alkoxy, Phenoxy, Benzyloxy,
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)-Alkylcarbonyloxy, (C₃-C₈)-Cycloalkylcarbonyloxy, Benzoyloxy, (C7-C₁₁)-Phenylalkylcarbonyloxy,
(C₁-C₈)-Alkoxycarbonyloxy, Phenoxycarbonyloxy, (C₇-C₁₁)-Phenalkyloxycarbonyl, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy-(C₁-C₄)-alkyl-carbamoyl, Acyloxy-(C₁-C₄)-alkyl-carbamoyl,
Carbamoyloxy, N-(C₁-C₆)-Alkylcarbamoyloxy, N,N-Di(C₁-C₆)-alkylcarbamoyloxy, N-(C₃-C₈)-Cycloalkylcarbamoyloxy,
R⁵ einen unverzweigten (C₁-C₄)-Alkylrest bedeutet, der mit einem Rest aus der Reihe Hydroxy, Carboxyl, (C₁-C₄)-Alkoxy, Phenoxy, Benzyloxy,
(C₁-C₉)-Alkoxycarbonyl, Phenoxycarbonyl, (C₇-C₁₁)-Phenylalkyloxycarbonyl, (C₃-C₈)-Cycloalkoxycarbonyl,
(C₁-C₁₂)-Alkylcarbonyloxy, (C₃-C₈)-Cycloalkylcarbonyloxy, Benzoyloxy, (C7-C₁₁)-Phenylalkylcarbonyloxy,
(C₁-C₈)-Alkoxycarbonyloxy, Phenoxycarbonyloxy, (C₇-C₁₁)-Phenylalkylcarbonyloxy, (C₃-C₈)-Cycloalkoxycarbonyloxy,
Carbamoyl, N-(C₁-C₆)-Alkylcarbamoyl, N,N-Di(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy-(C₁-C₄)-alkyl-carbamoyl, Acyloxy-(C₁-C₄)-alkyl-carbamoyl, (C₁-C₆)-Alkoxy-(C₁-C₆)-alkylcarbamoyl,
Carbamoyloxy, N-(C₁-C₆)-Alkylcarbamoyloxy, N,N-Di(C₁-C₆)alkylcarbamoyloxy, N-(C₃-C₈)-Cycloalkylcarbamoyloxy, oder mit
einem Rest der allgemeinen Formel III
-CO-P (III),
worin
P ein über seine terminale Aminogruppe gebundenes Di- oder Tripeptid oder ein über ihre Aminogruppe gebundenes Aminosäure-Derivat bedeutet, substituiert ist
f 1 bis 5
g 0,1 bis (2f+1) und
x 0 oder 1
bedeuten.

2. Verfahren zur Herstellung von Verbindungen nach Anspruch 1, in dem R⁵ bis R⁷ die Bedeutung gemäß des Anspruchs 1 aufweisen, dadurch gekennzeichnet, daß man Pyridin-2-carbonsäureestercarboxylate der Formel 7" 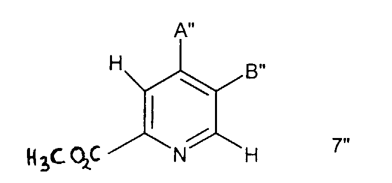 in der
A" = H und B" = = -CO₂H oder
A" = -CO₂H und B = H bedeuten,
mit einem Sulfonsäureamidderivat der Formel 9,
R⁶HN-R⁷ 9
und die hieraus entstehenden Verbindungen mit einem Amin der Formel 5
HNHR⁵ 5
zu den Verbindungen der Formel I umsetzt, 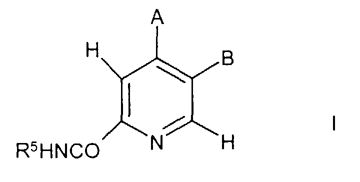 in denen
A = H und B = -CO-NR⁶-R⁷ oder
A = -CO-NR⁶-R⁷ und B = H bedeuten.

3. Verbindungen nach Anspruch 1 zur Anwendung gegen fibrotische Erkrankungen.

4. Verbindungen nach Anspruch 1 zur Anwendung als Fibrosuppressiva.

5. Arzneimittel, enthaltend mindestens eine Verbindung gemäß Anspruch 1 und ggf. einen pharmazeutisch verträglichen Träger.

6. Verbindungen gemäß Anspruch 1 zur Behandlung von Störungen des Stoffwechsels von Collagen und collagenähnlichen Stoffen.

7. Verfahren zur Herstellung von Arzneimitteln zur Behandlung von fibrotischen Erkrankungen, dadurch gekennzeichnet, daß das Arzneimittel eine Verbindung gemäß Anspruch 1 enthält.

## Claims

1. A sulfonamidocarbonylpyridine-2-carboxamide of the formula I 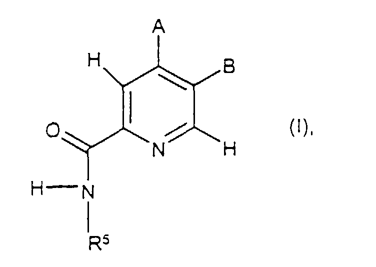 in which
A = H and B = -CO-NR⁶-R⁷ or
B = H and A = -CO-NR⁶-R⁷ and
R⁶ is hydrogen or a 1- or 2-valent physiologically utilizable cation,
R⁷ is a radical of the formula II
-SO₂-C-U-D-W (II),
in which
C is a bond or (C₁-C₄)-alkanediyl,
U is a bond or -O-,
D is a bond or (C₁-C₄)-alkanediyl,
W is a phenyl radical, U only having the meaning of -O- if C is not a bond or if D is not a bond and W is substituted by hydrogen, fluorine, chlorine, (C₁-C₆)-alkyl, (C₃-C₈)-cycloalkyl, (C₆-C₁₂)-aryl, (C₇-C₁₆)-aralkyl, (C₃-C₁₂)-alkenyl, (C₁-C₆)-alkoxy, phenoxy, -O-[-CH₂]ₓC_{f}H_{(2f+1-g})F_{g},
carbamoyl, N-(C₁-C₁₀)-alkylcarbamoyl, N,N-di-(C₁-C₈)-alkylcarbamoyl, N-(C₃-C₈)-cycloalkylcarbamoyl, N-phenylcarbamoyl, N-(C₇-C₁₁)-phenylalkylcarbamoyl, N-(C₁-C₈)-alkyl-N-phenylcarbamoyl, N-(C₁-C₈)-alkyl-N-(C₇-C₁₁)-phenylalkylcarbamoyl, N-(C₁-C₈)-alkyl-N-phenylalkylcarbamoyl,
N-((C₁-C₁₀)-alkoxy-(C₁-C₈)alkyl)carbamoyl, N-phenoxycarbamoyl, N-((C₇-C₁₆)-phenylalkyloxy-(C₁-C₈)-alkyl)carbamoyl, N-(C₁-C₈)-alkyl-N-((C₁-C₆)-alkoxy-(C₁-C₈)alkyl)carbamoyl, N-(C₁-C₈)-alkyl-N-(phenoxy-(C₁-C₈)alkyl)carbamoyl, N-(C₁-C₈)-alkyl-N-((C₇-C₁₆)phenylalkyloxy-(C₁-C₈)alkyl)carbamoyl,
(C₁-C₈)-alkanoylamino, (C₃-C₈)-cycloalkanoylamino, phenylamino, (C₇-C₁₁)-phenylalkanoylamino, (C₁-C₈)-alkanoyl-N-(C₁-C₁₀)-alkylamino, (C₃-C₈)-cycloalkanoyl-N-(C₁-C₆)-alkylamino, benzoyl-N-(C₁-C₁₀)-alkylamino, (C₇-C₁₁)-phenylalkanoyl-N-(C₁-C₆)-alkylamino, (C₁-C₁₀)-alkanoylamino-(C₁-C₈)-alkyl, (C₃-C₈)-cycloalkanoylamino-(C₁-C₈)-alkyl, phenylamino-(C₁-C₈)-alkyl, (C₇-C₁₁)-phenylalkanoylamino(C₁-C₈)-alkyl,
where the radicals which contain an aryl radical can for their part be substituted on the aryl by 1, 2 or 3 identical or different substituents from the group consisting of hydroxyl, carboxyl, (C₁-C₄)-alkoxy, phenoxy, benzyloxy,
(C₁-C₉)-alkoxycarbonyl, phenoxycarbonyl, (C₇-C₁₁)-phenylalkoxycarbonyl, (C₃-C₈)-cycloalkoxycarbonyl,
(C₁-C₁₂)-alkylcarbonyloxy, (C₃-C₈)-cycloalkylcarbonyloxy, benzoyloxy, (C₇-C₁₁)-phenylalkylcarbonyloxy,
(C₁-C₈)-alkoxycarbonyloxy, phenoxycarbonyloxy, (C₇-C₁₁)-phenalkyloxycarbonyl, (C₃-C₈)-cycloalkoxycarbonyloxy, carbamoyl, N-(C₁-C₆)-alkylcarbamoyl, N,N-di(C₁-C₆)-alkylcarbamoyl, N-(C₃-C₈-cycloalkylcarbamoyl, N-phenylcarbamoyl, N-(C₇-C₁₁)-phenylalkylcarbamoyl, hydroxy-(C₁-C₄)-alkylcarbamoyl, acyloxy-(C₁-C₄)-alkylcarbamoyl,
carbamoyloxy, N-(C₁-C₆)-alkylcarbamoyloxy, N,N-di-(C₁-C₆)-alkylcarbamoyloxy, N-(C₃-C₈)-cycloalkylcarbamoyloxy,
R⁵ is an unbranched (C₁-C₄)-alkyl radical which is substituted by a radical from the group consisting of hydroxyl, carboxyl, (C₁-C₄)-alkoxy, phenoxy, benzyloxy,
(C₁-C₉)-alkoxycarbonyl, phenoxycarbonyl, (C₇-C₁₁)-phenylalkyloxycarbonyl, (C₃-C₈)-cycloalkoxycarbonyl,
(C₁-C₁₂)-alkylcarbonyloxy, (C₃-C₈)-cycloalkylcarbonyloxy, benzoyloxy, (C₇-C₁₁)-phenylalkylcarbonyloxy,
(C₁-C₈)-alkoxycarbonyloxy, phenoxycarbonyloxy, (C₇-C₁₁)-phenylalkylcarbonyloxy, (C₃-C₈)-cycloalkoxycarbonyloxy, carbamoyl, N-(C₁-C₆)-alkylcarbamoyl, N,N-di(C₁-C₆)-alkylcarbamoyl, N-(C₃-C₈)-cycloalkylcarbamoyl, N-phenylcarbamoyl, N-(C₇-C₁₁)-phenylalkylcarbamoyl, hydroxy-(C₁-C₄)-alkylcarbamoyl, acyloxy-(C₁-C₄)-alkylcarbamoyl, (C₁-C₆)-alkoxy-(C₁-C₆)-alkylcarbamoyl, carbamoyloxy, N-(C₁-C₆)-alkylcarbamoyloxy, N,N-di-(C₁-C₆)-alkylcarbamoyloxy, N-(C₃-C₈)-cycloalkylcarbamoyloxy, or by
a radical of the formula III
-CO-P (III),
in which
P is a di- or tripeptide bonded via its terminal amino group or an amino acid derivative bonded via its amino group,
f is 1 to 5
g is 0.1 to (2f + 1) and
x is 0 or 1.

2. A process for the preparation of compounds as claimed in claim 1, in which R⁵ to R⁷ have the meaning as claimed in claim 1, which comprises reacting pyridine-2-carboxylic acid ester carboxylates of the formula 7" 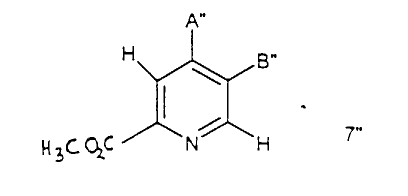 in which
A" = H and B" = -CO₂H or
A" = -CO₂H and B = H,
with a sulfonamide derivative of the formula 9
R⁶-HN-R⁷ 9
and reacting the compounds resulting therefrom with an amine of the formula 5
HNHR⁵ 5
to give the compounds of the formula I 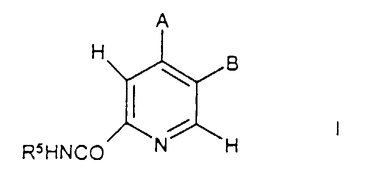 in which
A = H and B = -CO-NR⁶-R⁷ or
A = -CO-NR⁶-R⁷ and B = H.

3. A compound as claimed in claim 1 for use against fibrotic disorders.

4. A compound as claimed in claim 1 for use as a fibrosuppressant.

5. A pharmaceutical comprising at least one compound as claimed in claim 1 and, if appropriate, a pharmaceutically tolerable vehicle.

6. A compound as claimed in claim 1 for the treatment of disorders of the metabolism of collagen and collagen-like substances.

7. A process for the production of pharmaceuticals for the treatment of fibrotic disorders, wherein the pharmaceutical contains a compound as claimed in claim 1.

## Revendications

1. Sulfonamidocarbonylpyridine-2-carboxamides de formule générale I 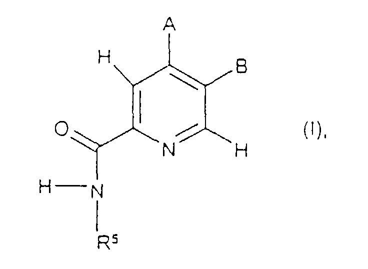 dans laquelle
A = H et B = -CO-NR⁶-R⁷ ou
B = H et A = -CO-NR⁶-R⁷ et
R⁶ représente un atome d'hydrogène ou un cation monovalent ou divalent physiologiquement utilisable,
R⁷ représente un radical de formule II
-SO₂-C-U-D-W (II)
dans laquelle
C représente une liaison ou un groupe alcanediyle en C₁-C₄,
U représente une liaison ou -O-,
D représente une liaison ou un groupe alcanediyle en C₁-C₄,
W représente le radical phényle, U n'ayant alors la signification de -O- que lorsque C ne représente pas une liaison ou lorsque D ne représente pas une liaison, et
W est substitué par un atome d'hydrogène, de fluor ou de chlore ou par un groupe alkyle en C₁-C₆, cycloalkyle en C₃-C₈, aryle en C₆-C₁₂, aralkyle en C₇-C₁₆, alcényle en C₃-C₁₂, alcoxy en C₁-C₆, phénoxy, -O-[-CH₂]ₓC_{f}H_{(2f+1-g})F_{g},
carbamoyle, N-alkyl(C₁-C₁₀)carbamoyle, N,N-dialkyl(C₁-C₈)carbamoyle, N-cycloalkyl(C₃-C₈)carbamoyle, N-phénylcarbamoyle, N-phénylalkyl(C₇-C₁₁)carbamoyle, N-alkyl(C₁-C₈)-N-phénylcarbamoyle, N-alkyl(C₁-C₈)-N-phénylalkyl(C₇-C₁₁)carbamoyle, N-alkyl(C₁-C₈)-N-phénylalkylcarbamoyle, N-(alcoxy(C₁-C₁₀)alkyl(C₁-C₈)carbamoyle, N-phénoxycarbamoyle, N-(phénylalkyl(C₇-C₁₆)oxy-alkyl(C₁-C₈))carbamoyle, N-alkyl(C₁-C₈)-N-alcoxy(C₁-C₆)alkyl(C₁-C₈)carbamoyle, N-alkyl(C₁-C₈)-N-(phénoxyalkyl(C₁-C₈))carbamoyle, N-alkyl(C₁-C₈)-N-(phénylalkyl(C₇-C₁₆)oxy-alkyl(C₁-C₈))carbamoyle,
alcanoyl(C₁-C₈)amino, cycloalcanoyl(C₃-C₈)amino, phénylamino, phénylalcanoyl(C₇-C₁₁)amino, alcanoyl(C₁-C₈)-N-alkyl(C₁-C₁₀)amino, cycloalcanoyl(C₃-C₈)-N-alkyl(C₁-C₆)amino, benzoyl-N-alkyl(C₁-C₁₀)amino, phénylalcanoyl(C₇-C₁₁)-N-alkyl(C₁-C₆)amino, alcanoyl(C₁-C₁₀)amino-alkyle(C₁-C₈), cycloalcanoyl(C₃-C₈)amino-alkyle(C₁-C₈), phénylamino-alkyle(C₁-C₈), phénylalcanoyl(C₇-C₁₁)aminoalkyle(C₁-C₈),
les radicaux qui contiennent un fragment aryle pouvant pour leur part porter sur le fragment aryle 1, 2 ou 3 substituants identiques ou différents, choisis parmi des groupes hydroxy, carboxy, alcoxy en C₁-C₄, phénoxy, benzyloxy,
alcoxy(C₁-C₉)carbonyle, phénoxycarbonyle, phénylalcoxy(C₇-C₁₁)carbonyle, cycloalcoxy(C₃-C₈)carbonyle,
alkyl(C₁-C₁₂)carbonyloxy, cycloalkyl(C₃-C₈)carbonyloxy, benzoyloxy, phénylalkyl(C₇-C₁₁)carbonyloxy,
alcoxy(C₁-C₈)carbonyloxy, phénoxycarbonyloxy, phénalkyloxy(C₇-C₁₁)carbonyle, cycloalcoxy(C₃-C₈)carbonyloxy,
carbamoyle, N-alkyl(C₁-C₆)carbamoyle, N,N-dialkyl(C₁-C₆)carbamoyle, N-cycloalkyl(C₃-C₈)carbamoyle, N-phénylcarbamoyle, N-phénylalkyl(C₇-C₁₁)carbamoyle, hydroxyalkyl(C₁-C₄)carbamoyle, acyloxy-alkyl(C₁-C₄)carbamoyle,
carbamoyloxy, N-alkyl(C₁-C₆)carbamoyloxy, N,N-dialkyl(C₁-C₆)carbamoyloxy, N-cycloalkyl(C₃-C₈)carbamoyloxy,
R⁵ représente un radical alkyle en C₁-C₄ non ramifié, qui est substitué par un radical choisi parmi un groupe hydroxy, carboxy, alcoxy en C₁-C₄, phénoxy, benzyloxy,
alcoxy(C₁-C₉)carbonyle, phénoxycarbonyle, phénylalkyl(C₇-C₁₁)oxycarbonyle, cycloalcoxy(C₃-C₈)carbonyle,
alkyl(C₁-C₁₂)carbonyloxy, cycloalkyl(C₃-C₈)carbonyloxy, benzoyloxy, phénylalkyl(C₇-C₁₁)carbonyloxy,
alcoxy(C₁-C₈)carbonyloxy, phénoxycarbonyloxy, phénylalkyl(C₇-C₁₁)carbonyloxy, cycloalcoxy(C₃-C₈)carbonyloxy, carbamoyle, N-alkyl(C₁-C₆)carbamoyle, N,N-dialkyl(C₁-C₆)carbamoyle, N-cycloalkyl(C₃-C₈)carbamoyle, N-phénylcarbamoyle, N-phénylalkyl(C₇-C₁₁)carbamoyle, hydroxyalkyl(C₁-C₄)carbamoyle, acyloxy-alkyl(C₁-C₄)carbamoyle, alcoxy(C₁-C₆)-alkyl(C₁-C₆)carbamoyle,
carbamoyloxy, N-alkyl(C₁-C₆)carbamoyloxy, N,N-dialkyl(C₁-C₆)carbamoyloxy, N-cycloalkyl(C₃-C₈)carbamoyloxy,
ou par un radical de formule générale III
-CO-P (III)
dans laquelle
P représente un di- ou tripeptide lié par son groupe amino terminal, ou un dérivé d'aminoacide lié par son groupe amino,
f va de 1 à 5,
g va de 0,1 à (2f+1) et
x est égal à 0 ou 1.

2. Procédé pour la préparation de composés selon la revendication 1, dans lesquels R⁵ à R⁷ ont les significations selon la revendication 1, caractérisé en ce que l'on fait réagir des pyridine-2-carboxylatoester-carboxylates de formule 7" 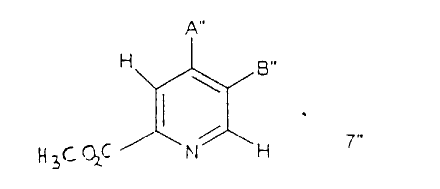 dans laquelle
A" = H et B" = = -CO₂H ou
A" = = -CO₂H et B = H,
avec un dérivé de sulfonamide de formule 9
R⁶HN-R⁷ 9
et on fait réagir les composés résultants avec une amine de formule 5
HNHR⁵ 5,
pour aboutir aux composés de formule I 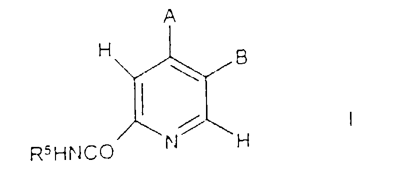 dans laquelle
A = H et B = -CO-NR⁶-R⁷ ou
A" = -CO-NR⁶-R⁷ et B = H.

3. Composés selon la revendication 1, pour utilisation contre des maladies fibreuses.

4. Composés selon la revendication 1, pour utilisation en tant que fibrosuppresseurs.

5. Médicament, contenant au moins un composé selon la revendication 1 et éventuellement un véhicule pharmaceutiquement acceptable.

6. Composés selon la revendication 1, pour le traitement de troubles du métabolisme du collagène et de substances de type collagène.

7. Procédé pour la fabrication de médicaments destinés au traitement de maladies fibreuses, caractérisé en ce que le médicament contient un composé selon la revendication 1.
